(19) Europäisches Patentamt
European Patent Office
Office européen des brevets

(11) Publication number: **0 132 392 B1**

(12) # EUROPEAN PATENT SPECIFICATION

(45) Date of publication of patent specification: **02.01.92**

(51) Int. Cl.$^5$: **C07B 53/00**, C07B 43/08, C07B 41/02, C07C 255/00, C07D 307/54, C07D 333/24, C07D 209/48, C07D 209/52, C07D 213/04, C07K 5/12

(21) Application number: **84304951.1**

(22) Date of filing: **19.07.84**

Divisional application 88114033 filed on 29.08.88.

(54) **Asymmetric synthesis of alpha-substituted-alpha-cyanomethyl alcohols.**

(30) Priority: **22.07.83 AU 432/83**
**08.12.83 AU 2758/83**

(43) Date of publication of application:
**30.01.85 Bulletin 85/05**

(45) Publication of the grant of the patent:
**02.01.92 Bulletin 92/01**

(84) Designated Contracting States:
**AT BE CH DE FR IT LI LU NL SE**

(56) References cited:
**CH-A- 589 051**
**GB-A- 2 013 670**
**US-A- 4 163 787**

**DIE MAKROMOLECULARE CHEMIE, vol. 183, no. 3, March 12, 1982, Basel, Heidelberg, New York; J. OKU et al.: "Asymmetric Cyanohydrin Synthesis Catalyzed by Synthetic Dipeptides, 2a)", page 579-586**

(73) Proprietor: **ICI AUSTRALIA LIMITED**
**1 Nicholson Street**
**Melbourne Victoria 3001(AU)**

(72) Inventor: **Jackson, William Roy**
**6 Freeman Street**
**Glen Waverley 3150(AU)**
Inventor: **Wilshire, Colin**
**7 Harry Street**
**Doncaster East, 3109(AU)**
Inventor: **Matthews, Barry Ross**
**6 Kenny Street**
**North Balwyn 3104(AU)**

(74) Representative: **Bishop, Nigel Douglas et al**
**Imperial Chemical Industries PLC Legal Department: Patents PO Box 6 Bessemer Road**
**Welwyn Garden City Herts, AL7 1HD(GB)**

**EP 0 132 392 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for the asymmetric synthesis of α -substituted- α -cyanomethyl alcohols and the use of such alcohols as intermediates in the preparation of chiral esters and chiral ethanolamines and in particular chiral pyrethroids and chiral arylethanolamines.

It is known in the art that in pyrethroids of general formula

```
    O    CN
    "    '
J-C-O-CH-R1,
          *
```

for a given pyrethrin acid or pyrethroid acid moiety JCO- and a given pyrethroid alcohol moiety $R^1$-CH(CN)-O-, there is a wide variation in pesticidal activity between the two pyrethroid isomers prepared from the two alcohol enantioners (* indicates a chiral centre). Therefore, there is considerable interest in the art in the development of methods for preparing the individual pyrethroid alcohol enantiomers and their use in the preparation of the more pesticidally active pyrethroid isomer.

A preferred method for preparing the individual pyrethroid alcohol enantiomers would be a synthesis which gave an enantiomer in substantially pure form. However, hitherto there has been no publication of an asymmetric synthesis of individual pyrethroid alcohol enantiomers. All methods for the preparation of individual pyrethroid alcohol enantiomers which have been described hitherto have involved the resolution of the racemic mixture of alcohols or derivatives thereof. For example, in United Kingdom Patent Application No 2 013 670A there is disclosed a process for the preparation of the pyrethroid alcohol (S)- α -cyano-3-phenoxybenzyl alcohol. The preparation involves a resolution process in which the racemic alcohol is reacted with an optically active lactone to give a mixture of the two diastereomeric ethers of the alcohol enantiomers, the diastereomeric ethers are separated by chromatography over silica gel and the ether of the (S)-alcohol is hydrolysed under acid conditions to give (S)- α -cyano-3-phenoxybenzyl alcohol which is purified by chromatography over silica gel. Clearly this is a long and costly process which, although suitable for small scale laboratory preparations, would prove difficult to adapt for large scale commercial manufacture.

It is known in the art that the naturally occurring physiologically active arylethanolamine norepinephrine (noradrenaline) is in the laevorotatory optically active form which has the absolute configuration (R) at the chiral centre. Many synthetic arylethanolamines have been prepared which are physiologically active by virtue of binding at norepinephrine receptor sites but to date all of their syntheses have involved the preparation of the racemic arylethanolamines.

In view of the difficulty in resolving the racemic mixtures of the sympathomimetic arylethanolamines, the commercially available pharmaceutical preparations containing these compounds have to date generally contained the racemic arylethanolamine. However, it has been found that S-isomer or dextro-rotatory optical isomer of these compounds may have some undesired effects. Therefore, clearly it would be advantageous to be able to synthesise the R-isomer of these arylethanolamines to give products more like the naturally occurring norepinephrine and hence products having a more selective action, a greater therapeutic ratio and more cost-effectiveness.

In a recent paper Ohu, Ito and Inoue (Makromol. Chem. 183, 579-586 (1982) disclosed a process for the asymmetric synthesis of cyanohydrins using synthetic dipeptides. In this paper there is described the reaction of benzaldehyde with hydrogen cyanide in the presence of a synthetic dipeptide to give α -cyanobenzyl alcohol. The paper reports that in the early stage of the reaction the addition was highly stereo-specific but the product isolated comprised a mixture of enantiomers with an enantiomeric excess only of the order of 0.1 to 10.1%. The conclusion drawn in the paper was that the catalyst racemized the product and that the process would be suitable for asymmetric cyanohydrin synthesis "if a procedure is established to separate the product rapidly from the catalyst before the catalysed racemization takes place".

It has now been found that α -substituted α -cyanomethyl alcohol enantiomers can be prepared in a highly stereospecific reaction by the asymmetric addition of hydrogen cyanide to aldehydes in the presence of a cyclic dipeptide catalyst without rapid separation of the product from the catalyst and that the product α -substituted- α -cyanomethyl alcohols comprise either substantially one enantiomer or a high proportion (typically 75 to 100%; ie 50 to 100% enantiomeric excess) of one enantiomer which may be used in the preparation of pyrethroid enantiomers and arylethanolamine enantiomers.

It is to be understood that in the context of this specification the term "enantiomer" is used to refer to a product comprising substantially one enantiomer or a product comprising a high proportion, typically 75% or more and preferably more than 90%, of one enantiomer.

Accordingly the invention provides a process for the preparation of an α -substituted- α -cyanomethyl alcohol enantiomer of formula I

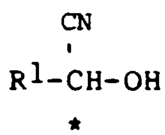

I

which comprises reacting an aldehyde of formula II

$R^1$-CHO II

with hydrogen cyanide in the presence of a cyclic dipeptide enantiomer, and wherein said reaction is carried out at a temperature below ambient temperature, wherein $R^1$ is selected from:

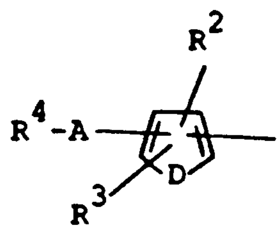

III

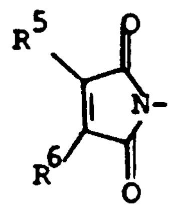

IV

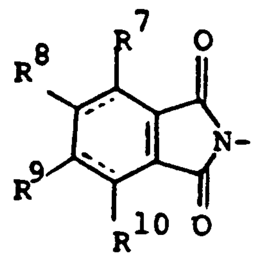

V

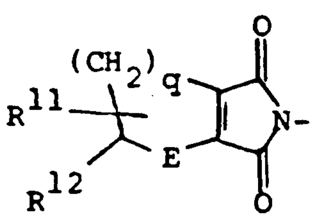

VI

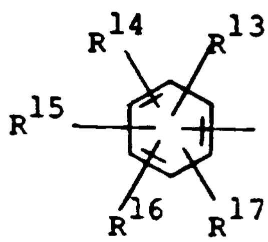

VII

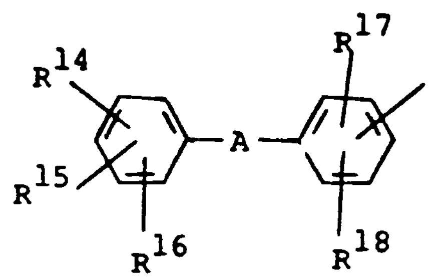

VIII

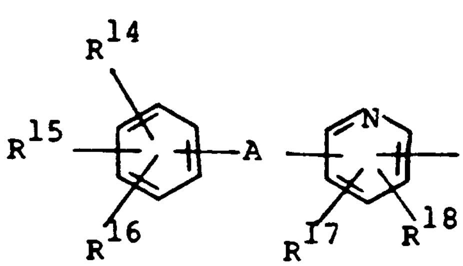

IX

$R^{20}R^{21}C{=}C(R^{19})-$

X

$$R^{22}CH_2-C=C-$$

XI

wherein:

$R^2$ and $R^3$ are independently selected from hydrogen, halogen, $C_1$ to $C_6$ alkyl and $C_1$ to $C_6$ haloalkyl, or $R^2$ and $R^3$ jointly form a trimethylene or tetramethylene bridging group;

A is selected from oxygen, sulfur, -CO- and $-CH_2-$;

$R^4$ is selected from hydrogen, $C_1$ to $C_6$ alkyl, $C_2$ to $C_6$ alkenyl, $C_2$ to $C_6$ alkynyl, phenyl, furyl, thienyl and the groups phenyl, furyl and thienyl wherein each group is substituted by halogen, $C_1$ to $C_6$ alkyl, $C_1$ to $C_6$ alkoxy or $C_2$ to $C_6$ alkenyl;

D is selected from oxygen and sulfur;

$R^5$ and $R^6$ are independently selected from $C_1$ to $C_6$ alkyl;

$R^7$, $R^8$, $R^9$, $R^{10}$ are independently selected from hydrogen, halogen, and $C_1$ to $C_6$ alkyl;

$R^{11}$ and $R^{12}$ are independently selected from hydrogen, $C_1$ to $C_6$ alkyl and halogen, or $R^{11}$ and $R^{12}$ jointly form a methylenedioxy bridging group;

E is selected from oxygen, sulfur and $-CH_2-$;

q is an integer selected from 1 and 2;

$R^{13}$ is selected from hydrogen,

halogen, $C_1$ to $C_6$ alkyl, $C_2$ to $C_6$ alkenyl, $C_2$ to $C_6$ alkynyl, thienyloxy, thenyl, furylmethyl and the groups $R^{24}R^{25}C=C(R^{23})0$- and $R^{24}R^{25}C=C(R^{23})$- in which $R^{23}$ is selected from hydrogen and $C_1$ to $C_6$ alkyl and $R^{24}$ and $R^{25}$ are independently selected from hydrogen, halogen, $C_1$ to $C_6$ alkyl and $C_1$ to $C_6$ haloalkyl;

$R^{14}$, $R^{15}$, $R^{16}$, $R^{17}$ and $R^{18}$ are independently selected from the group consisting of hydrogen, $C_1$ to $C_6$ alkyl, halogen and $C_2$ to $C_6$ alkenyl;

$R^{19}$ is selected from hydrogen, chlorine and $C_1$ to $C_6$ alkyl;

$R^{20}$ and $R^{21}$ are independently selected from hydrogen, fluorine, chlorine, bromine, $C_1$ to $C_6$ alkyl, $C_2$ to $C_6$ alkenyl, $C_2$ to $C_6$ alkynyl, phenyl, benzyl, furylmethyl and thienylmethyl; and

$R^{22}$ is selected from phenyl, phenoxy and the groups phenyl and phenoxy wherein each group is substituted by halogen, nitro, cyano, $C_1$ to $C_6$ alkyl, $C_1$ to $C_6$ halo-alkyl or $C_1$ to $C_6$ alkoxy

and wherein said cyclic dipeptide enantiomer comprises the residues of two amino acids selected from alanine, cysteine, histidine, homoserine, isoleucine, leucine, lysine, methionine, norleucine, norvaline, ornithine, phenylalanine, serine, thyronine, tryptophan, tyrosine, valine, and the N-alkyl, N-alkenyl and N-acyl derivatives thereof.

Preferred values for $R^1$ include groups of formulae III, IV, V, VI, VII, VIII, IX, X and XI wherein:

$R^2$ and $R^3$ are independently selected from hydrogen, halogen, $C_1$ to $C_6$ alkyl and $C_1$ to $C_6$ haloalkyl, or $R^2$ and $R^3$ jointly form a trimethylene or tetramethylene bridging group;

A is selected from oxygen, sulfur, -CO- and $-CH_2-$;

$R^4$ is selected from hydrogen, $C_1$ to $C_6$ alkyl, $C_2$ to $C_6$ alkenyl, $C_2$ to $C_6$ alkynyl, phenyl, furyl, thienyl and the groups phenyl, furyl and thienyl wherein each group is substituted by halogen, $C_1$ to $C_6$ alkyl, $C_1$ to $C_6$ alkoxy or $C_2$ to $C_6$ alkenyl;

D is selected from oxygen and sulfur;

$R^5$ and $R^6$ are independently selected from $C_1$ to $C_6$ alkyl;

$R^7$, $R^8$, $R^9$ and $R^{10}$ are independently selected from hydrogen, halogen, and $C_1$ to $C_6$ alkyl;

$R^{11}$ and $R^{12}$ are independently selected from hydrogen, $C_1$ to $C_6$ alkyl and halogen, or $R^{11}$ and $R^{12}$ jointly form a methylenedioxy bridging group;

E is selected from oxygen, sulfur and $-CH_2-$;

q is an integer selected from 1 and 2;

$R^{13}$, $R^{14}$, $R^{15}$, $R^{16}$, $R^{17}$ and $R^{18}$ are independently selected from: hydrogen; halogen; nitro; $C_1$ to $C_6$ alkyl; $C_1$ to $C_6$ haloalkyl; $C_1$ to $C_6$ hydroxyalkyl; $C_2$ to $C_6$ alkenyl; $C_2$ to $C_6$ alkynyl; $C_1$ to $C_6$ alkoxy; $C_1$ to $C_6$ alkylthio; ($C_1$ to $C_6$ alkoxy) carbonyl; benzyloxy, substituted benzyloxy; acyloxy; hydroxy; tri($C_1$ to $C_6$ alkyl)-silyloxy; ($C_1$ to $C_6$ alkoxy) $C_1$ to $C_6$ alkoxy;$C_1$ to $C_6$ alkoxy-$C_1$ to $C_6$ alkoxymethoxy; amino; N-($C_1$ to $C_6$ alkyl)amino; N,N-di($C_1$ to $C_6$ alkyl)amino; N-($C_1$ to $C_6$ alkanoyl)amino; N-($C_1$ to $C_6$ alkylsulfonyl)-amino; N-(benzenesulfonyl)amino; N-(substituted benzene-sulfonyl)amino; ureido; N-[tri($C_1$ to $C_6$ alkyl)silyl]amino; sulfamoyl; N-($C_1$ to $C_6$ alkyl)sulfamoyl; N,N-di($C_1$ to $C_6$ alkyl)-sulfamoyl; carbamoyl; N-($C_1$ to $C_6$ alkyl)-carbamoyl; N,N-di($C_1$ to $C_6$ alkyl)carbamoyl; $C_1$ to $C_6$ alkylsulfinyl; $C_1$ to $C_6$ alkylsulfonyl; thienyloxy; thenyl; furylmethyl; or two adjacent substituents are selected from the linking group buta-1,3-dienylene; and the groups $R^{24}R^{25}C=C(R^{23})O$- and $R^{24}R^{25}C=C(R^{23})$- in which $R^{23}$ is selected from hydrogen and $C_1$ to $C_6$

alkyl and $R^{24}$ and $R^{25}$ are independently selected from hydrogen, halogen, $C_1$ to $C_6$ alkyl and $C_1$ to $C_6$ haloalkyl;
$R^{19}$ is selected from hydrogen, chlorine and $C_1$ to $C_6$ alkyl;
$R^{20}$ and $R^{21}$ are independently selected from hydrogen, fluorine, chlorine, bromine, $C_1$ to $C_6$ alkyl, $C_2$ to $C_6$ alkenyl, $C_2$ to $C_6$ alkynyl, phenyl, benzyl, furylmethyl and thienylmethyl; and
$R^{22}$ is selected from phenyl, phenoxy and the groups phenyl and phenoxy wherein each group is substituted by halogen, nitro, cyano, $C_1$ to $C_6$ alkyl, $C_1$ to $C_6$ halo-alkyl or $C_1$ to $C_6$ alkoxy.

When $R^1$ is a group of formula VIIb and one or more of $R^{13}$, $R^{14}$, $R^{15}$, $R^{16}$ and $R^{17}$ are selected from acyl, suitable acyl groups include $C_2$ to $C_6$ alkanoyl, benzoyl and substituted benzoyl.

When $R^1$ is a group of formula VIIb and one or more of $R^{13}$, $R^{14}$, $R^{15}$, $R^{16}$ and $R^{17}$ are selected from substituted benzyloxy, substituted benzoyl or N-(substituted benzenesulfonyl)amino, suitable benzene ring substituents include one to three substituents selected from the group consisting of halogen, nitro, cyano, $C_1$ to $C_6$ alkyl, $C_1$ to $C_6$ haloalkyl, $C_1$ to $C_6$ alkoxy and $C_1$ to $C_6$ alkylthio.

More preferred values for $R^1$ include groups of formulae III, VII, VIII and IX above and in particular groups of formulae

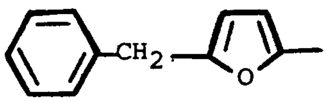

IIIa

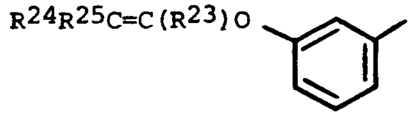

VIIa

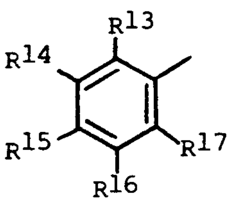

VIIb

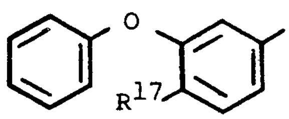

VIIIa

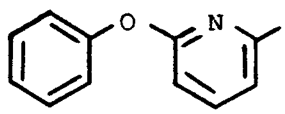

IXa

wherein:
in formula VIIIa, $R^{17}$ is selected from hydrogen and halogen;
in formula VIIb, $R^{13}$, $R^{14}$, $R^{15}$, $R^{16}$ and $R^{17}$ are independently selected from hydrogen, halogen, nitro, hydroxy, $C_1$ to $C_6$ alkyl, $C_1$ to $C_6$ haloalkyl, $C_1$ to $C_6$ hydroxyalkyl, $C_1$ to $C_6$ alkoxy, ($C_1$ to $C_6$ alkoxy)$C_1$ to $C_6$ alkoxy, amino, N-($C_2$ to $C_6$ alkanoyl)amino, N-($C_1$ to $C_6$ alkylsulfonyl) amino, sulfamoyl, ureido, benzyloxy, benzoyloxy, or two adjacent substituents are selected from the linking group buta-1,3-dienylene;
and in formula VIIa, $R^{23}$ is hydrogen and $R^{24}$ and $R^{25}$ are independently selected from hydrogen and halogen.

Even more preferred values for $R^1$ include groups of formulae IIIa, VIIa, VIIb and VIIIa wherein; in formula VIIIa, $R^{17}$ is selected from hydrogen and fluorine;
in formula VIIb, $R^{13}$, $R^{14}$, $R^{15}$, $R^{16}$ and $R^{17}$ are independently selected from hydrogen, halogen, methyl, trifluoromethyl, hydroxymethyl, methoxy, 1-(ethoxy)ethoxy, nitro, amino, acetamido, methanesulfonylamino, sulfamoyl, ureido, benzyloxy, benzoyloxy, or two adjacent substituents are selected from the linking group buta-1,3-dienylene;
in formula VIIa, $R^{23}$ is hydrogen and $R^{24}$ and $R^{25}$ are independently selected from hydrogen and halogen.

Specific examples of the compounds of formula I which may be prepared according to the process of the present invention include both the (R)- and (S)-enantiomers of the compounds illustrated in Tables 1 and 2 below.

TABLE 1

| | |
|---|---|
| 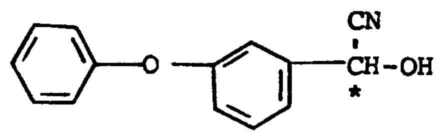 | 1 |
| 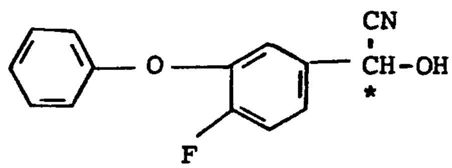 | 2 |
| 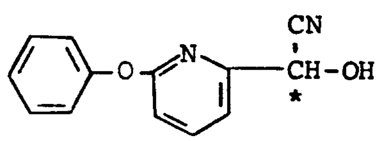 | 3 |
| 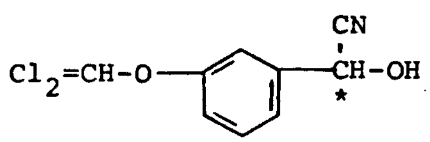 | 4 |
| 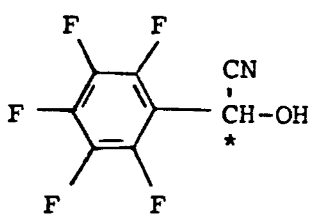 | 5 |
| 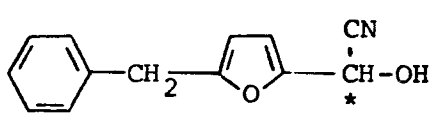 | 6 |

TABLE 2

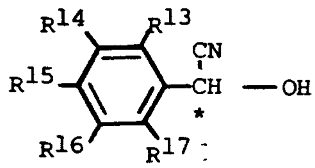

| Compound | Substituents | | | | |
|---|---|---|---|---|---|
| No | $R^{13}$ | $R^{14}$ | $R^{15}$ | $R^{16}$ | $R^{17}$ |
| 8 | H | H | H | H | H |
| 9 | H | HO | H | H | H |
| 10 | H | HO | HO | H | H |
| 11 | H | HO | H | HO | H |
| 12 | H | $HOCH_2$ | HO | H | H |
| 13 | H | $CH_2SO_2NH$ | HO | H | H |
| 14 | H | H | HO | H | H |
| 15 | $H_2NCONH$ | HO | H | H | H |
| 16 | H | Cl | $H_2N$ | Cl | H |
| 17 | Cl | H | H | H | H |
| 18 | H | $CH_3O$ | HO | $CH_3O$ | H |
| 19 | H | $H_2NSO_2$ | $CH_3$ | H | H |
| 20 | H | H | $NO_2$ | H | H |
| 21 | H | $CH_3O$ | H | H | H |
| 22 | H | H | $CH_3O$ | H | H |
| 23 | H | H | $CF_3$ | H | H |
| 24 | H | -CH=CH-CH=CH- | | H | H |
| 25 | H | Cl | H | H | H |
| 26 | H | $CH_3CO$ | H | H | H |
| 27 | H | $NO_2$ | H | H | H |
| 28 | H | a | a | H | H |
| 29 | H | a | H | H | H |
| 30 | H | H | Cl | H | H |
| 31 | H | H | b | H | H |
| 32 | H | c | c | H | H |
| 33 | H | d | d | H | H |
| 34 | H | $CH_3O$ | $CH_3O$ | H | H |
| 35 | $CH_3$ | H | H | H | H |

Code:

a - $C_6H_5CH_2O$

b - $CH_3CONH$

c - $C_6H_5COO$

d - $CH_3CH_2OCH(CH_3)O$

One particularly preferred compound which may be made is high yield and high enantiomeric excess according to the process of the present invention is the (S)-isomer of compound No 1, that is (S)- α -cyano-3-phenoxybenzyl alcohol.

One particularly preferred group of compounds which may be made according to the process of the present invention are the (R)-isomers of compounds of formula I in which $R^1$ has the formula VIIb

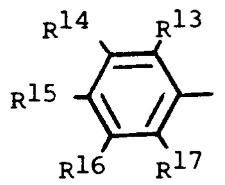

VIIb

wherein;
from one to three of the substituents $R^{13}$, $R^{14}$, $R^{15}$, $R^{16}$ and $R^{17}$ are selected from methoxy, methyl, halogen, hydroxy, nitro, trifluoromethyl, acetyloxy and benzyloxy, or two adjacent substituents are selected from the linking group buta-1,3-dienylene; and
the remaining substituents are hydrogen.

In the preparation, according to the process of the present invention, of compounds of formula I in which $R^1$ is a group of formula VII, VIII or IX wherein one or more of $R^{13}$, $R^{14}$, $R^{15}$, $R^{16}$ or $R^{17}$ is a basic or acidic substituent, for example hydroxy or amino, or a substituent with a strong electronic effect, for example nitro or sulfamoyl, such substituents may adversely affect the rate and/or stereospecificity of the reaction. However, compounds of formula I containing such substituents may be readily prepared according to the process of the present invention by masking or protecting such interferring substituents utilizing masking or protecting groups of the type well known in the art. The process of the present invention may be effected on the compound of formula II having masking or protecting groups and the protecting groups removed from the product to afford the desired compound of formula I.

Suitable masking or protecting groups:
for hydroxy include $C_2$ to $C_6$ carboxyl esters, benzoyl and substituted benzoyl esters, $C_1$ to $C_6$ alkyl ethers, benzyl and substituted benzyl ethers, trialkylsilyl ethers, and acetals or ketals formed with $C_1$ to $C_6$ aldehydes or $C_3$ to $C_6$ ketones and $C_1$ to $C_6$ alcohols; for amino and N-($C_1$ to $C_6$ alkyl)amino, include $C_2$ to $C_6$ alkanoyl amides, benzoyl and substituted benzoyl amides, $C_1$ to $C_6$ alkyl sulfonyl amides, benzenesulfonyl and substituted benzenesulfonyl amides and trialkylsilyl amides;
for carboxy include $C_1$ to $C_6$ alkyl esters and benzyl and substituted benzyl esters;
for nitro include amides, sulfonamides and silyl amides as indicated above for amino;
for sulfamoyl, N-($C_1$ to $C_6$ alkyl)sulfamoyl and N,N-di ($C_1$ to $C_6$ alkyl)sulfamoyl include thioethers which may be oxidized and reacted with the appropriate amine; and for carbamoyl, N-($C_1$ to $C_6$ alkyl)carbamoyl and N,N-di ($C_1$ to $C_6$ alkyl)carbamoyl, include esters, as indicated above for carboxy, which may be reacted with the appropriate amine.

It is to be understood that the process of the present invention includes within its scope the preparation of compounds of formula I having masked or protected substituents by reaction of corresponding compounds of formula II having masked or protected substituents and release of the desired compound of formula I if desired by removal of the masking or protecting group.

Suitable cyclic dipeptide enantiomers which may be used as catalysts in the process of the present invention include diketopiperazines of the formula

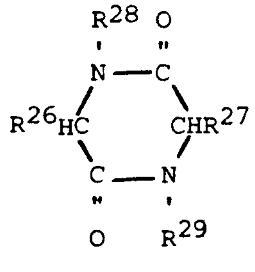

XII

which may be prepared by coupling two α -amino acids of formula XIII and XIV wherein $R^{28}$ and $R^{29}$ are hydrogen or by coupling two α -amino acid derivatives of formula XIII and XIV wherein $R^{28}$ and $R^{29}$ are substituents other than hydrogen.

$R^{26}CH(NHR^{28})COOH$ XIII

$R^{27}CH(NHR^{29})COOH$ XIV

Suitable amino acids include alanine, cysteine, histidine, homoserine, isoleucine, leucine, lysine, methionine, norleucine, norvaline, ornithine, phenylalanine, serine, thyronine, tryptophan, tyrosine, valine and the N-alkyl, N-alkenyl and N-acyl derivatives thereof. Preferably one of the amino acids in the diketopiperazine of formula XII is chosen from histidine, tryptophan or a derivative thereof. Preferably the other amino acid in the diketopiperazine of formula XII is one which has a large or bulky α -substituent. Examples of such amino acids include histidine, phenylalanine, thyronine, tyrosine, tryptophan and derivatives thereof.

Examples of diketo-piperazine derivatives include compounds of formula XII wherein $R^{26}$ and $R^{27}$ are independently selected from phenyl, benzyl, 4-hydroxybenzyl, 4-benzyloxybenzyl, 4-methoxybenzyl, 4-phenyl, 4-methyl, 4-imidazolylmethyl and 3-indolylmethyl, and $R^{28}$ and $R^{29}$ are independently selected from hydrogen, alkyl, alkenyl and acyl. Such compounds include, for example: (R)-3-benzyl-(R)-6-(4-imidazolylmethyl)-2,5-piperazinedione (cyclic (R)-phenylalanyl-(R)-histidine; C.(R)-Phe-(R)-His); (S)-3-benzyl-(S)-6-(4-imidazolylmethyl)-2,5-piperazinedione (cyclic (S)-phenylalanyl-(S)-histidine; C.(S)-Phe-(S)-His); (S)-3-benzyl-(R)-6-(4-imidazolylmethyl)-2,5-piperazinedione (cyclic (S)-phenylalanyl-(R)-histidine; C.(S)-Phe-(R)-His); (R)-3-(4-hydroxybenzyl)-(R)-6-(4-imidazolylmethyl)-2,5-piperazinedione (cyclic (R)-tyrosyl-(R)-histidine; C.(R)-Tyr-(R)-His); (R)-3-(4-benzyloxybenzyl)-(R)-6-(4-imidazolylmethyl)-2,5-piperazinedione (cyclic O-benzyl-(R)-tyrosyl-(R)-histidine; C.O-BZ-(R)-Tyr-(R)-His); (R)-3-(4-methoxybenzyl)-(R)-6-(4-imidazolylmethyl)-2,5-piperazinedione (cyclic O-methyl-(R)-tyrosyl-(R)-histidine; C.O-Me-(R)-Tyr-(R)-His); (R)-4-phenyl-(R)-6-(4-imidazolylmethyl)-2,5-piperazinedione (cyclic (R)-phenylglycyl-(R)-histidine; C.(R)-Phegly-(R)-His); (R)-4-methyl-(R)-6-(4-imidazolylmethyl)-2,5-piperazinedione (cyclic (R)-alanyl-(R)-histidine; C.(R)-Ala-(R)-His); (S),-(S)-3,6-bis(4-imidazolylmethyl)-2,5-piperazinedione(cyclic (S)-histidyl-(S)-histidine; C.(S)-His-(S)-His); and (R),(R)-3,6-bis(4-imidazolylmethyl)-2,5-piperazinedione (cyclic (R)-histidyl-(R)-histidine; C.(R)-His-(R)-His).

Preferred cyclic dipeptide enantiomers for use in the process of the present invention include (R)-3-benzyl-(R)-6-(4-imidazolylmethyl)-2,5-piperazinedione (C.(R)-Phe-(R)-His) and (S)-3-benzyl-(S)-6-(4-imidazolylmethyl)-2,5-piperazinedione (C.(S)-Phe-(S)-His).

Certain of the cyclic dipeptides which may be used as catalysts in the process of the present invention are novel compounds. For example, certain cyclic dipeptides in which (R)-histidine is one of the amino acid residues and which have been found to be particularly suitable for the preparation of the (S)-isomers of α -substituted- α -cyanomethyl alcohols according to the process of the present invention are novel compounds.

Accordingly in a further embodiment the invention provides a cyclic dipeptide enantiomer comprising (R)-histidine or a derivative thereof as one of the amino acid residues.

The cyclic dipeptide enantiomers of this embodiment of the present invention are diketopiperazines which may be depicted by the following formula XXXVI

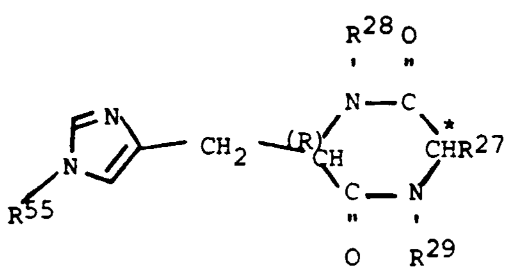

XXXVI

and which may be prepared by the coupling of the amino acid (R)-histidine, or a derivative thereof, of formula XXXVII

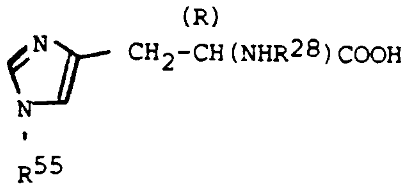

XXXVII

and an amino acid, or a derivative thereof, of formula XXXVIII

$$R^{27}-\underset{*}{C}H(NHR^{29})COOH \qquad XXXVIII$$

Suitable (R)-histidines of formula XXXVII include (R)-histidine and the N-alkyl, N-alkenyl and N-acyl derivatives thereof. That is, for example, compounds of formula XXXVII wherein $R^{28}$ and $R^{55}$ are independently selected from hydrogen, $C_1$ to $C_6$ alkyl, $C_2$ to $C_6$ alkenyl, $C_2$ to $C_6$ alkanoyl and benzoyl. Preferably the amino acid of formula XXXVII is (R)-histidine.

Suitable amino acids of formula XXXVIII include alanine, cysteine, histidine, homoserine, isoleucine, leucine, lysine, methionine, norleucine, norvaline, ornithine, phenylalanine, serine, thyronine, tryptophan, tyrosine, valine and the N-alkyl, N-alkenyl and N-acyl derivatives thereof. Preferably the amino acid of formula XXXVIII is one which has a large or bulky $\alpha$ -substituent. Such amino acids include, for example, histidine, phenylalanine, thyronine, tyrosine, tryptophan and derivatives thereof. Phenylalanine is a preferred amino acid of formula XXXVIII.

It is preferred that the amino acids comprising the cyclic dipeptide enantiomer of this embodiment of the present invention have the same stereochemistry. Therefore, the preferred cyclic dipeptide enantiomers comprise (R)-histidine and another (R)-amino acid. A more preferred cyclic dipeptide enantiomer comprises (R)-histidine and (R)-phenylalanine, that is, the compound of formula XXXVI in which $R^{27}$ is benzyl, $R^{28}$, $R^{29}$ and $R^{55}$ are each hydrogen and the asymmetric centre alpha to $R^{27}$ has the (R)-configuration.

The cyclic dipeptide enantiomers of formulae XII and XXXVI may be prepared by standard methods known to those skilled in the art. For example, the cyclic dipeptide enantiomers of the invention may be made by classical solution synthesis by the coupling of suitably protected (R)-histidine with another suitably protected amino acid. The cyclic dipeptide enantiomer (R)-3-benzyl-(R)-6(4-imidazolylmethyl)2,5-piperazinedione may be prepared, for example, by coupling N-benzyloxycarbonyl-(R)-phenylalanine and (R)-histidine methyl ester in the presence of dicyclhexylcarbodiimide and benzotriazole to give N-benzyloxycarbonyl-(R)-phenylalanyl-(R)-histidine methyl ester, removal of the N-benzyloxycarbonyl protecting group by hydrogenation and cyclization.

The cyclic dipeptide enantiomers of formulae XII and XXXVI are, in general, crystalline solids and if they are crystallized from a solvent comprising water and/or an alcohol they may be in the form of hydrates or alcoholates and/or they may contain water or alcohol of crystallization.

In the process of the present invention the reaction between the aldehyde of formula II and hydrogen cyanide, in the presence of a cyclic dipeptide enantiomer catalyst, is preferably carried out in the presence of a solvent. The nature of the solvent is not narrowly critical but preferably it is a solvent in which the aldehyde of formula II and hydrogen cyanide are soluble. The cyclic dipeptide enantiomer catalyst may be soluble in the solvent, homogeneous catalysis, insoluble in the solvent, heterogeneous catalysis, or adsorb the solvent swelling up to form a gel. Preferred solvents include inert hydrocarbons, halocarbons and halohydrocarbons which are solvents for the aldehyde of formula II and hydrogen cyanide and which are adsorbed by the catalyst to give a gel. Preferred solvents include the aromatic hydrocarbons such as, for example benzene, toluene and the xylenes.

One of the distinct advantages of the process of the present invention is that the cyclic dipeptide enantiomers of formulae XII and XXXVI may be recovered from the reaction mixture and reused. The cyclic dipeptide enantiomers may be recovered by treating the reaction mixture, or the residue after removal of the solvent from the reaction mixture, with a solvent, for example diethyl ether, in which the reaction product is soluble but in which the cyclic dipeptide enantiomer catalyst is insoluble. The catalyst may then be recovered by filtration, recrystallised from water or an aqueous solvent, and dried.

The amount of the cyclic dipeptide enantiomer used as a catalyst in the process of the present invention is not narrowly critical but preferably falls within the range of from $10^{-1}$ to $10^{-6}$ moles per mole of

the aldehyde of formula II.

Surprisingly, it has been found that the degree of hydration in the cyclic dipeptide enantiomers of formulae XII and XXXVI is important to the effectiveness of the catalyst in the process of the present invention. Preferably the catalyst has a degree of hydration which enables it to swell in the solvent being used as reaction medium to form a gel. Catalysts which have too little or too much water of hydration apparently fail to swell in the reaction medium and do not efficiently catalyse the asymmetric addition of hydrogen cyanide to the aldehydes of formula II. In practice, it has been found preferable if the catalyst contains between 0.5 and 1.5 moles, and more preferably 1 mole, of water of hydration or crystallization per mole of cyclic dipeptide enantiomer.

Also surprisingly, it has been found that the cyclic dipeptide enantiomers of formulae XII and XXXVI improve in stereospecificity of addition of hydrogen cyanide to aldehydes of formula II after their first use. Therefore preferably, the cyclic dipeptide enantiomers of formula XII and XXXVI have first been used, recovered, recrystallized and dried to the preferred hydration level before use in the process of the present invention to prepare α -substituted- α -cyanomethyl alcohol enantiomers of formula I.

In the process of the present invention the reaction between the aldehyde of formula II and hydrogen cyanide in the presence of a cyclic dipeptide enantiomer catalyst is carried out at a temperature below ambient temperature in order to obtain the desired stereospecificity of addition. Preferably the temperature is within the range from -20 to 10 °C, more preferably at or below 0 °C, and most preferably at or below -5 °C.

The reaction time required for the process of the present invention depends to a large extent on the specific aldehyde of formula II, the specific catalyst and the solvent used. However, in general a reaction time of between 1 and 100 hours is suitable and a reaction time of between 10 and 30 hours is preferred.

In the process of the present invention it has been found that cyclic dipeptide enantiomers made from (R)-amino acids catalyse the formation of (S)-α -substituted- α -cyanomethyl alcohol derivatives when the cyano group has a higher priority (according to the Cahn-Ingold-Prelog Rules c.f. R S Cahn, C K Ingold and V Prelog Angew Chem Int Ed (1966) 5, 385 or Comprehensive Organic Chemistry Vol 1 pp 16-18, Ed. Barton & Ollis, Pergamon Press 1979, ISBN O-08-021313-8) than the α -substitutent or conversely the stereochemically equivalent (R)-configuration when the cyano group has a lower priority than the α -substituent. Conversely, it has been found that the cyclic dipeptide enantiomers made from (S)-amino acids catalyse the formation of (R)- α -substituted-α -cyanomethyl alcohol derivatives when the cyano group has higher Cahn-Ingold-Prelog priority than the α -substituent or conversely the stereochemically equivalent (S)-configuration when the cyano group has a lower priority than the α -substituent. Therefore, the process of the present invention is eminently suitable for the preparation of either (R)- or (S)-α-substituted- α -cyanomethyl alcohol derivatives of formula I.

Certain of the compounds of formula I prepared according to the process of the present invention are novel compounds. Therefore, in yet a further embodiment the invention provides a compound of formula I wherein $R^1$ is a group of formula VIIb wherein $R^{13}$, $R^{14}$, $R^{15}$, $R^{16}$ and $R^{17}$ are as hereinbefore defined with the proviso that at least one of $R^{13}$, $R^{14}$, $R^{15}$, $R^{16}$ and $R^{17}$ is a substituent other than hydrogen.

Certain of the compounds of formula I are useful intermediates for the preparation of a range of pyrethroid pesticides of formula XV

```
  O   CN
  "   '
J-C-O-CH-R1                XV
```

wherein

```
  O
  "
J-C-O
```

is a pyrethrin acid moiety or a pyrethroid acid moiety.

Suitable J include groups of the formulae:

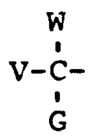

XVI

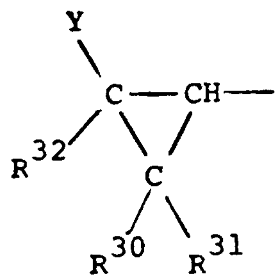

XVII

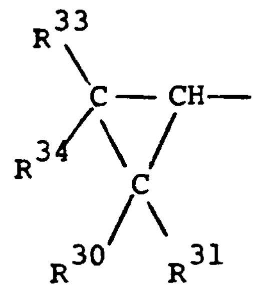

XVIII

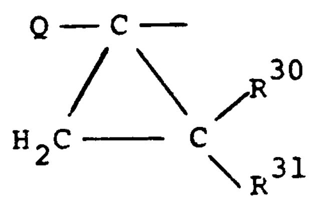

XIX

wherein:

V represents a substituted aromatic group or an unsaturated alicyclic group or an alkenyl group or an arylamino group and is selected from the group consisting of the formulae

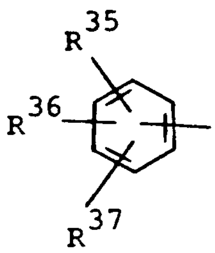

XX

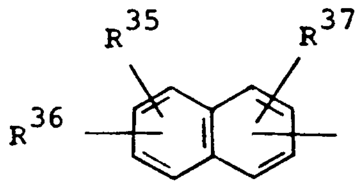

XXI

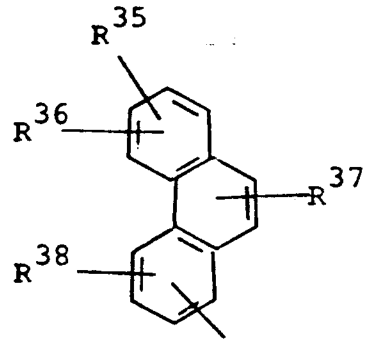

XXII

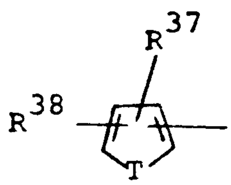

XXIII

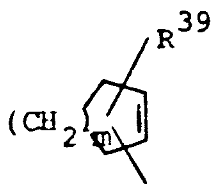

XXIV

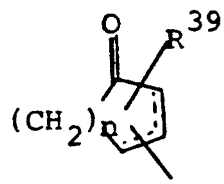

XXV

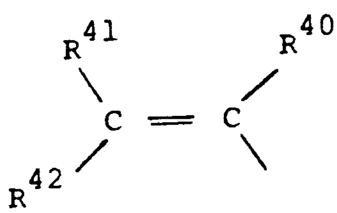

XXVI

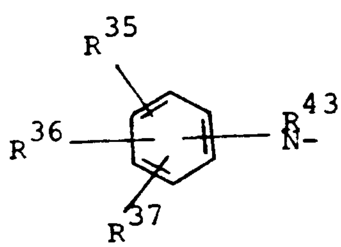

XXVII

wherein:

$R^{35}$ and $R^{36}$ are independently selected from hydrogen, halogen, cyano, nitro, $C_1$ to $C_6$ alkyl, $C_1$ to $C_6$ haloalkyl, ($C_1$ to $C_6$ alkoxy)$C_1$ to $C_6$ alkyl, $C_2$ to $C_6$ alkenyl, $C_2$ to $C_6$ haloalkenyl, $C_2$ to $C_6$ alkynyl, $C_2$ to $C_6$ haloalkynyl, $C_1$ to $C_6$ alkoxy, $C_1$ to $C_6$ alkylthio, $C_1$ to $C_6$ alkylsulfinyl, acyl, acyloxy, ($C_1$ to $C_6$ alkoxy)-carbonyl, ($C_2$ to $C_6$ alkenyloxy)carbonyl, ($C_2$ to $C_6$ alkynyloxy)carbonyl, or $R^{35}$ and $R^{36}$ may jointly form a methylenedioxy, tetramethylene or trimethylene group;

$R^{37}$ and $R^{38}$ are independently selected from hydrogen, halogen, cyano, nitro, $C_1$ to $C_6$ alkyl, $C_1$ to $C_6$ haloalkyl, ($C_1$ to $C_6$ alkoxy)$C_1$ to $C_6$ alkyl, $C_2$ to $C_6$ alkenyl, $C_2$ to $C_6$ haloalkenyl, $C_2$ to $C_6$ alkynyl, $C_2$ to $C_6$ haloalkynyl, $C_1$ to $C_6$ alkoxy, $C_1$ to $C_6$ alkylthio, $C_1$ to $C_6$ alkylsulfinyl, acyl, acyloxy, ($C_1$ to $C_6$ alkoxy)-carbonyl, ($C_2$ to $C_6$ alkenyloxy)carbonyl, and ($C_2$ to $C_6$ alkynyloxy)carbonyl;

T is selected from oxygen and sulfur;

$R^{39}$ is selected from hydrogen, halogen, cyano, nitro and $C_1$ to $C_6$ alkyl;

m and n are independently selected from the integers 1 to 3;

the dotted line in formula XXV represents a double bond present at a position either conjugated with or non-conjugated with the ketone group (C = O);

$R^{40}$, $R^{41}$ and $R^{42}$ are independently selected from hydrogen, $C_1$ to $C_6$ alkyl, $C_2$ to $C_6$ alkenyl, $C_2$ to $C_6$ alkynyl, halogen, acyl and acyloxy;

$R^{43}$ is selected from hydrogen and $C_1$ to $C_6$ alkyl;

W is selected from $C_1$ to $C_6$ alkyl, $C_1$ to $C_6$ haloalkyl, $C_2$ to $C_6$ alkenyl, $C_2$ to $C_6$ haloalkenyl, $C_2$ to $C_6$ alkynyl, $C_2$ to $C_6$ haloalkynyl, $C_1$ to $C_6$ alkoxy, cyano and $C_3$ to $C_7$ cycloalkyl;

G is selected from hydrogen and fluorine;
Y is selected from the groups $R^{45}R^{46}C=CR^{44}$- and $R^{45}R^{46}R^{48}C$-$CR^{44}R^{47}$- wherein:
$R^{44}$ is selected from hydrogen and $C_1$ to $C_6$ alkyl;
$R^{45}$ is selected from hydrogen, halogen, $C_1$ to $C_6$ alkyl and $C_1$ to $C_6$ haloalkyl;
$R^{46}$ is selected from hydrogen, halogen, $C_1$ to $C_6$ alkyl, $C_1$ to $C_6$ haloalkyl, ($C_1$ to $C_6$ alkoxy)$C_1$ to $C_6$ alkyl, ($C_2$ to $C_6$ alkenyloxy)$C_1$ to $C_6$ alkyl, ($C_2$ to $C_6$ alkynyloxy)$C_1$ to $C_6$ alkyl, ($C_1$ to $C_6$ alkoxy)carbonyl, acyl, phenyl, phenyl substituted by halogen, nitro, cyano, $C_1$ to $C_6$ alkyl, $C_1$ to $C_6$ haloalkyl, $C_1$ to $C_6$ alkoxy or $C_1$ to $C_6$ haloalkoxy, a substituent of the formula $R^{50}R^{51}C=CR^{49}$- wherein $R^{49}$, $R^{50}$ and $R^{51}$ are individually selected from hydrogen and $C_1$ to $C_6$ alkyl, and a substituent of the formula $R^{52}ON=CH$-wherein $R^{52}$ is selected from hydrogen, $C_1$ to $C_6$ alkyl, $C_2$ to $C_6$ alkenyl and $C_2$ to $C_6$ alkynyl; or
$R^{45}$ and $R^{46}$ jointly form a cyclic group of formula XXVIII

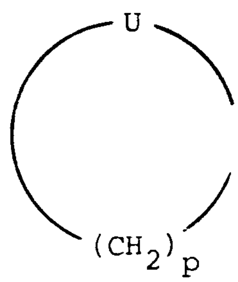

XXVIII

wherein U is selected from the group -$CH_2$-,

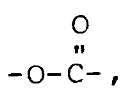 , 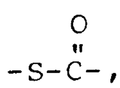 , 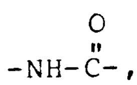 , 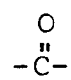

and p is an integer selected from 2 to 5; and
$R^{47}$ and $R^{48}$ are independently selected from hydrogen and halogen;
$R^{32}$ is selected from hydrogen and $C_1$ to $C_6$ alkyl;
$R^{30}$ and $R^{31}$ are independently selected from hydrogen, halogen and $C_1$ to $C_6$ alkyl or $R^{30}$ and $R^{31}$ jointly form an ethylene, trimethylene, tetramethylene or pentamethylene bridging group;
$R^{33}$ and $R^{34}$ are independently selected from hydrogen, $C_1$ to $C_6$ alkyl, halogen, phenyl, phenyl substituted by halogen, nitro, cyano, $C_1$ to $C_6$ alkyl, $C_1$ to $C_6$ haloalkyl or $C_1$ to $C_6$ alkoxy, or $R^{33}$ and $R^{34}$ jointly form a bridging group selected from ethylene, trimethylene, tetramethylene, pentamethylene and groups of the formulae

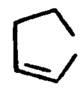

XXIX

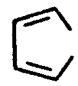

XXX

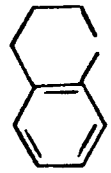

XXXI

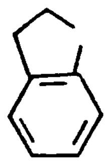

XXXII

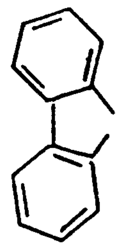

XXXIII

Q is selected from $C_1$ to $C_6$ alkyl, $C_2$ to $C_6$ alkenyl, $C_2$ to $C_6$ alkynyl and the group

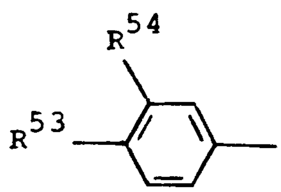

XXXIV

wherein:

$R^{53}$ is selected from hydrogen, $C_1$ to $C_3$ alkoxy, $C_1$ to $C_3$ alkylthio, $C_1$ to $C_2$ alkyl, nitro, fluoro, chloro, bromo and amino;

$R^{54}$ is selected from hydrogen and methyl; or

$R^{53}$ and $R^{54}$ jointly form a methylenedioxy bridging group.

Examples of specific groups of formula J include those groups illustrated in Table 3 below:

TABLE 3

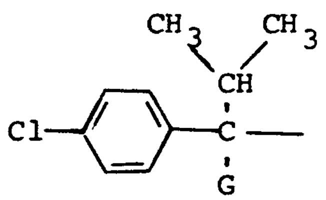

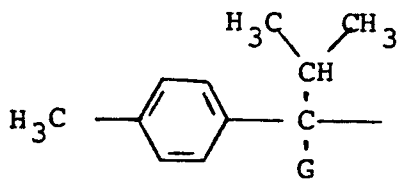

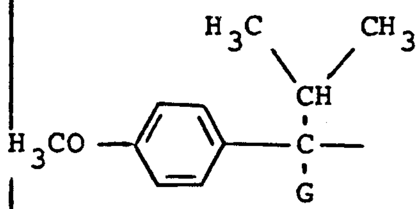

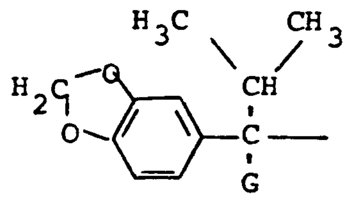

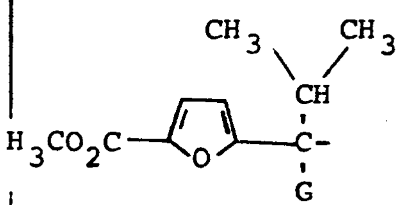

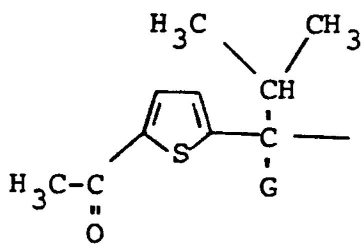

TABLE 3 - continued

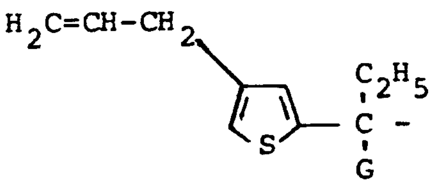
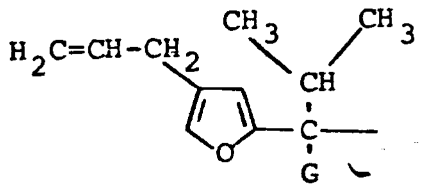
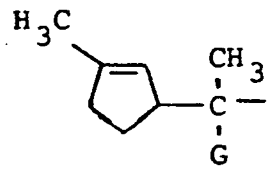
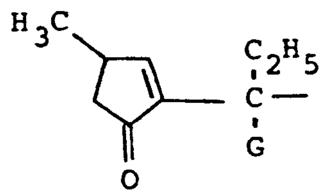
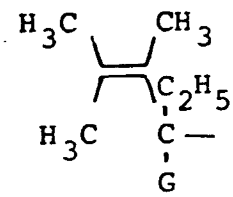
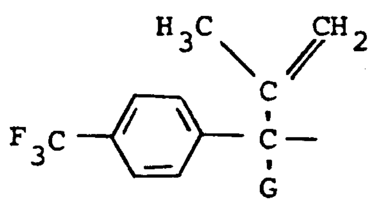
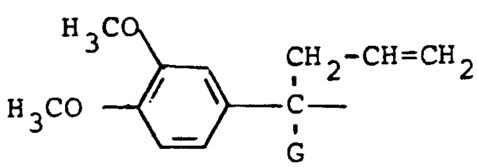
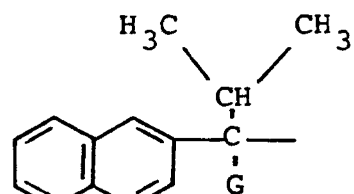
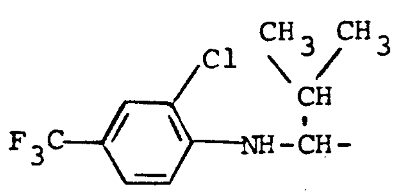
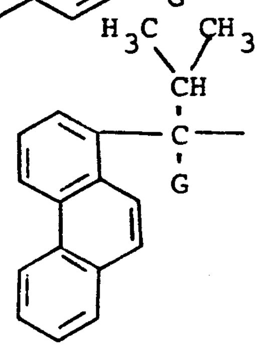
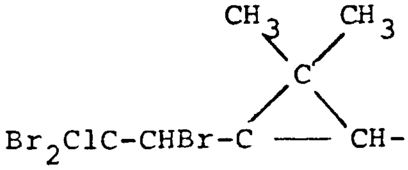
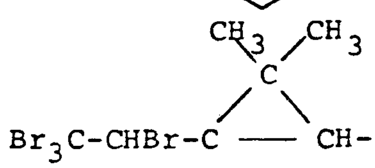

TABLE 3 - continued

| | |
|---|---|
| 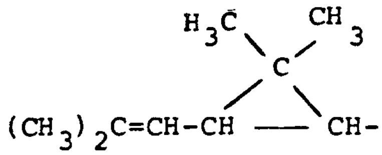 | 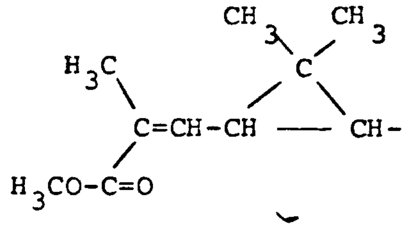 |
| 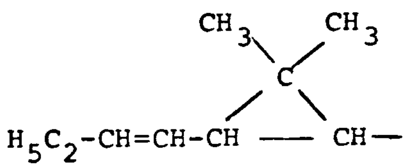 | 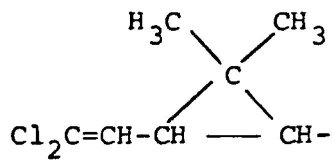 |
| 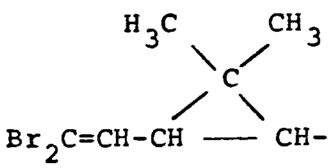 | 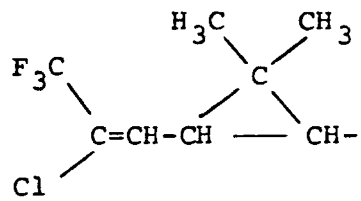 |
| 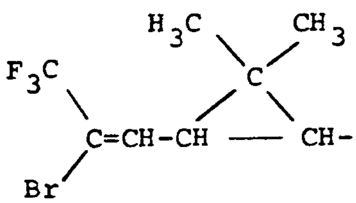 | 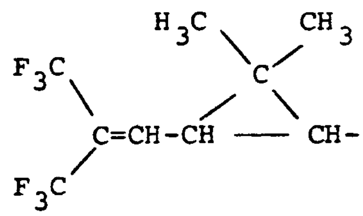 |
| 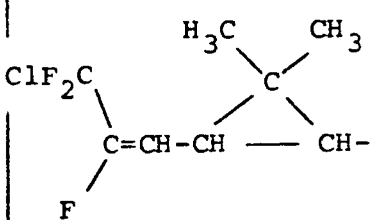 | 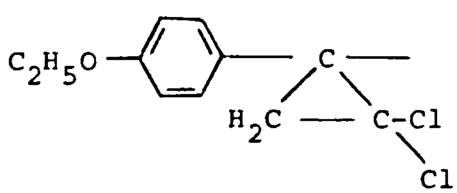 |
| 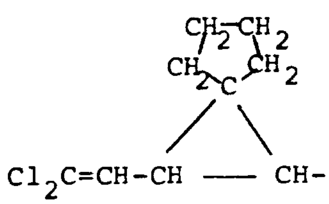 | 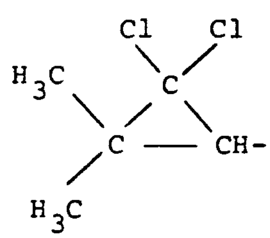 |

TABLE 3 - continued

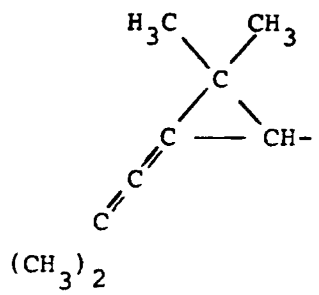

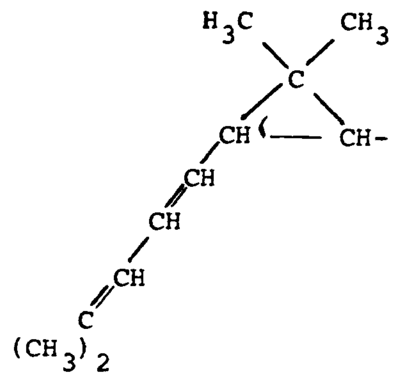

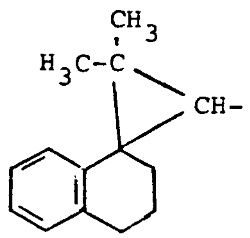

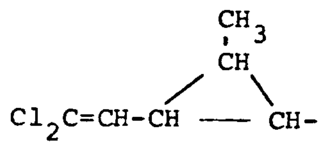

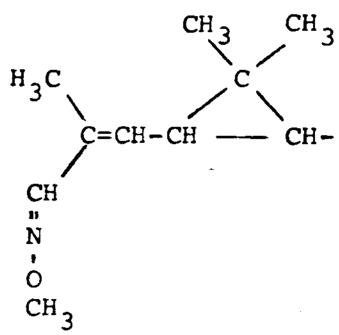

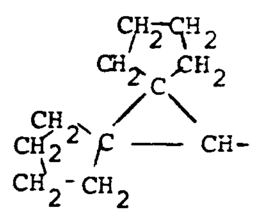

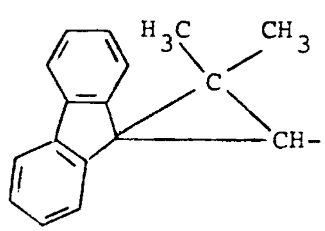

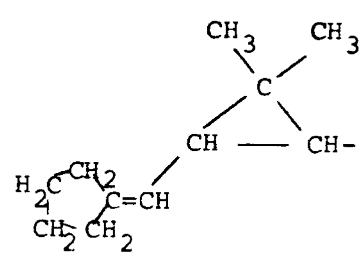

Pyrethroids of formula XV may be prepared from the compounds of formula I by esterification.

Accordingly in a further aspect, the invention provides a process for the preparation of a compound of formula XV which process comprises the esterification of an $\alpha$ -substituted- $\alpha$ -cyanomethyl alcohol enantiomer of formula I prepared as hereinbefore described, or a derivative thereof, with a pyrethrin acid or pyrethroid acid of formula XXXV

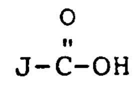

XXXV

wherein J is as hereinbefore defined, or a derivative thereof.

Preferably the α -substituted- α -cyanomethyl alcohol enantiomer of formula I prepared as hereinbefore described is selected from the group consisting of the enantiomers of:
α -cyano-3-phenoxybenzyl alcohol, α -cyano-4-fluoro-3-phenoxybenzyl alcohol, 3-(2,2-dichlorovinyloxy)- α -cyanobenzyl alcohol, α -cyano-pentafluorobenzyl alcohol, α -cyano- α -(6-phenoxypyrid-2-yl)methylalcohol and α -cyano- α -(5-benzylfur-2-yl)methylalcohol, that is compounds no 1, 2, 4, 5, 3 and 6 respectively.

The esterification may involve the reaction of a pyrethrin acid, pyrethroid acid or derivative thereof, such as the corresponding acid halide, with an α -substituted- α -cyanomethyl alcohol enantiomer, with retention of configuration of the pyrethroid alcohol enantiomer, for example as shown below

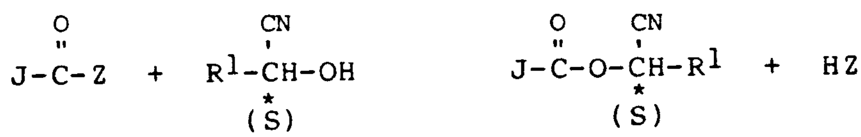

Alternatively, the esterification may involve the reaction of a pyrethrin acid, pyrethroid acid or derivative thereof, such as the corresponding alkali metal salt, with an α -substituted- α -cyanomethyl alcohol derivative, for example a tosyl, mesyl or benzenesulfonyl derivative, with inversion of configuration of the pyrethroid alcohol enantiomer, for example as shown below

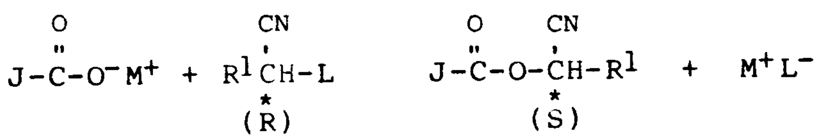

Specific examples of pyrethroids of formula XV which may be prepared from α -substituted- α -cyanomethylalcohols of formula I prepared according to the process of the present invention include those compounds illustrated in Table 4 below.

TABLE 4

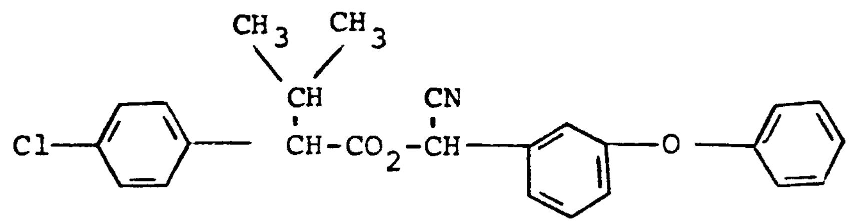

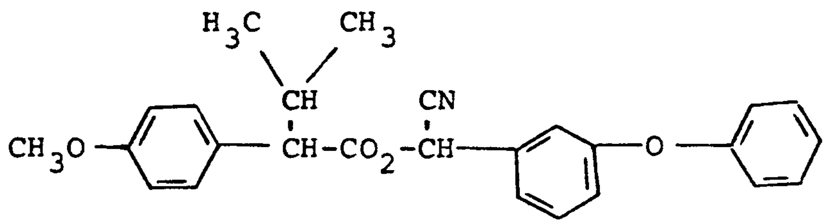

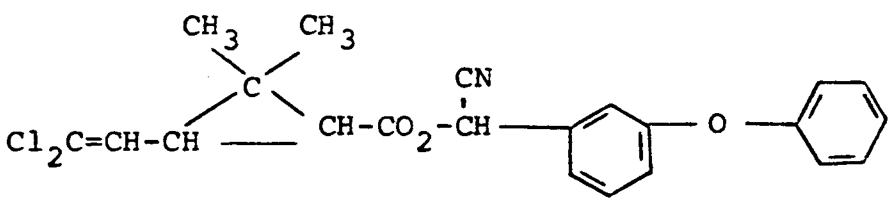

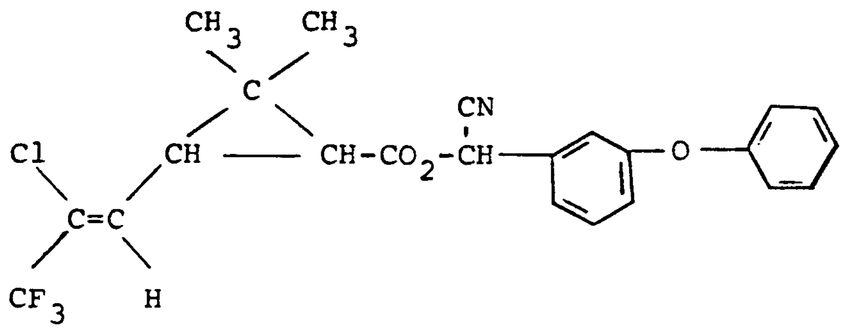

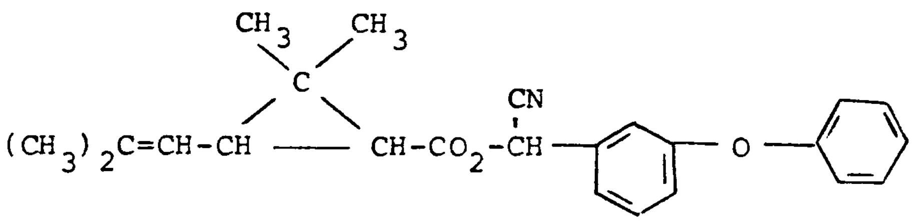

TABLE 4 - continued

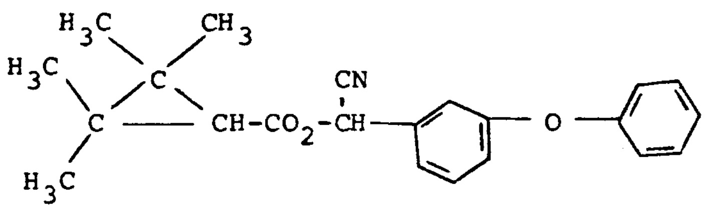

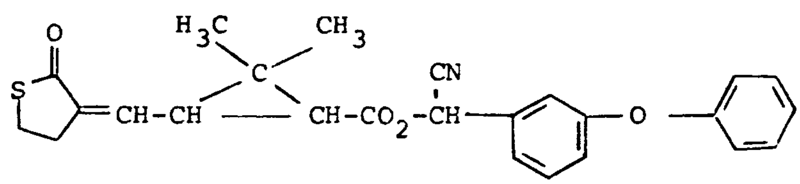

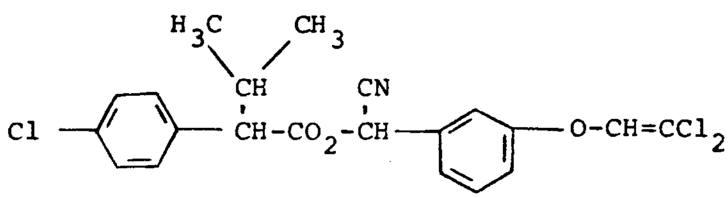

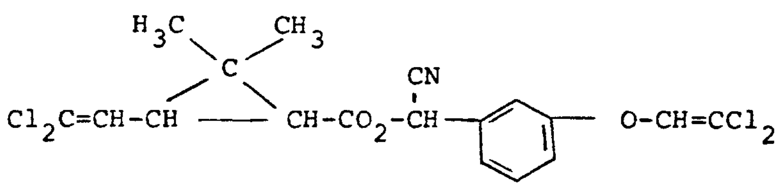

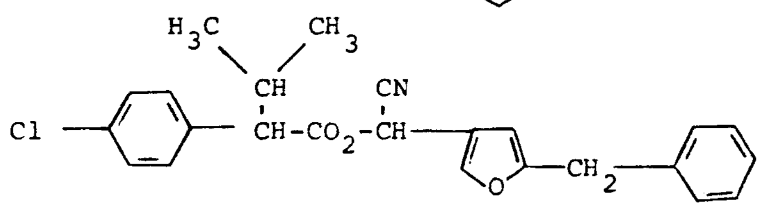

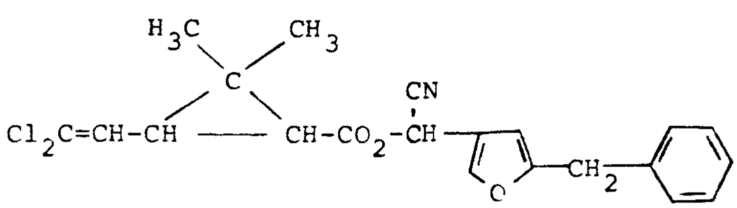

TABLE 4 - continued

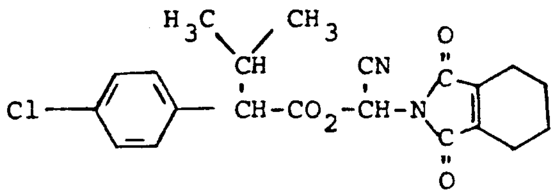

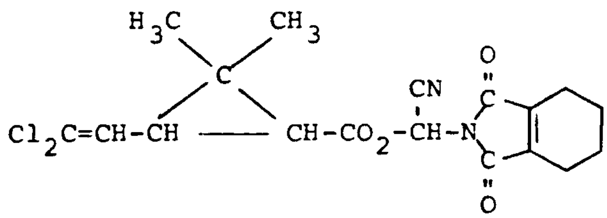

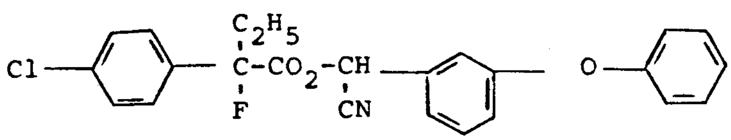

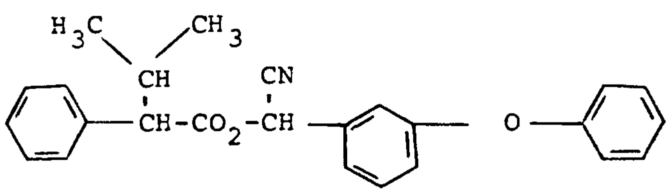

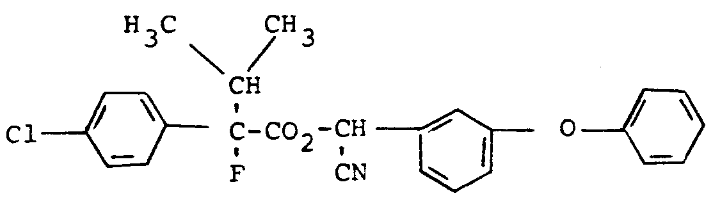

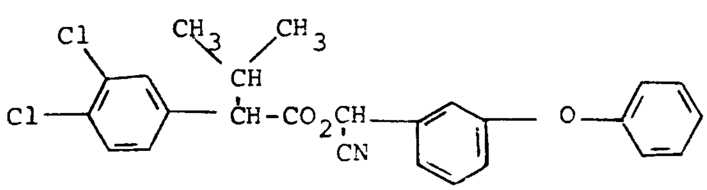

TABLE 4 - continued

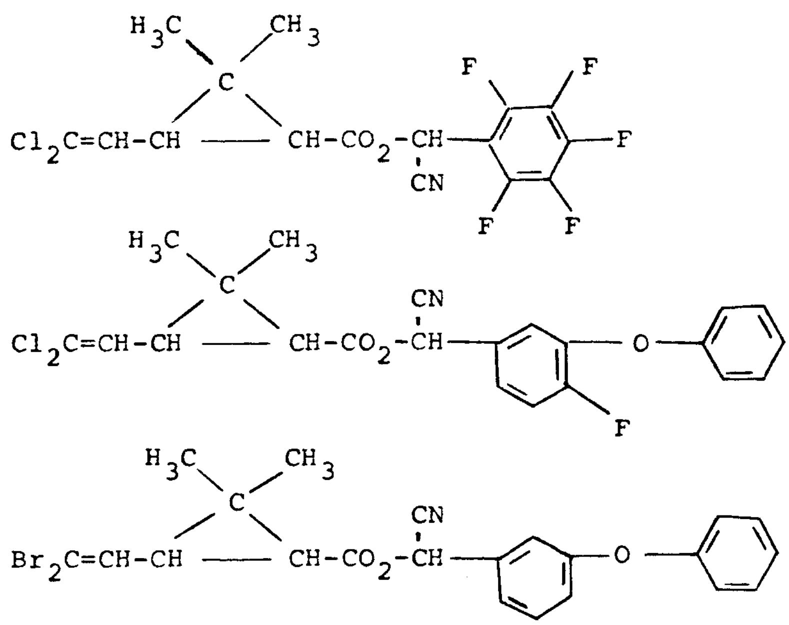

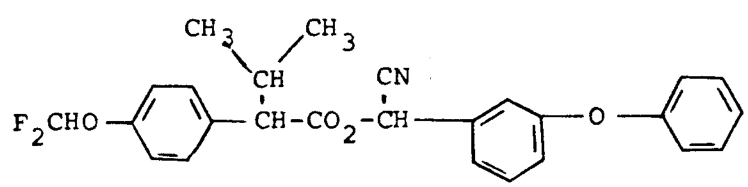

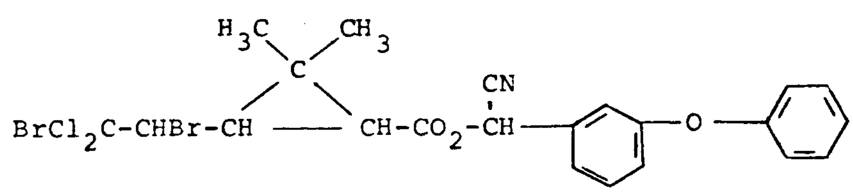

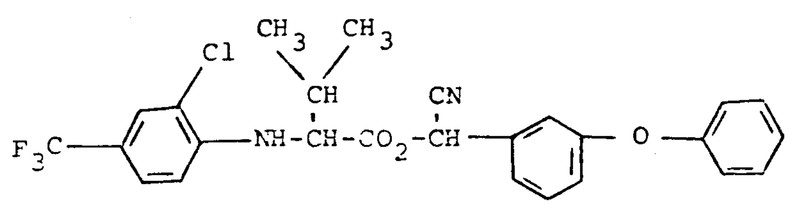

TABLE 4 - continued

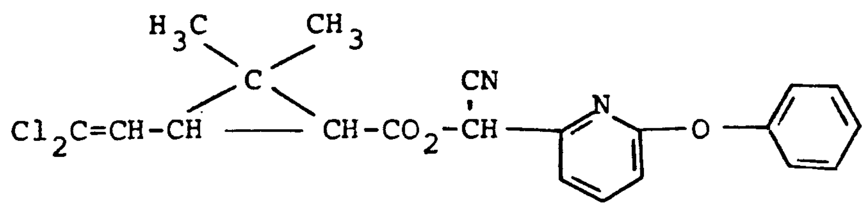

Certain of the compounds of formula I are useful intermediates for the preparation of arylethanolamine enantiomers of formula XXXIX

$$R^1-\underset{*}{\overset{OH}{C}}H-CH_2-NR^{56}R^{57} \qquad XXXIX$$

wherein:
one of $R^{56}$ and $R^{57}$ is selected from hydrogen and the other is selected from hydrogen, $C_1$ to $C_6$ alkyl and substituted $C_1$ to $C_6$ alkyl, or $R^{56}$ and $R^{57}$ together form a $C_4$ to $C_6$ alkylene linking group.

Among the preferred arylethanolamine enantiomers of formula XXXIX are those prepared from the novel compounds of formula I in which $R^1$ is a group of formula VIIb wherein $R^{13}$, $R^{14}$, $R^{15}$, $R^{16}$ and $R^{17}$ are as hereinbefore defined with the proviso that at least one of $R^{13}$, $R^{14}$, $R^{15}$, $R^{16}$ and $R^{17}$ is a substituent other than hydrogen.

Examples of suitable values for $R^{56}$ and $R^{57}$ include: hydrogen; $C_1$ to $C_6$ alkyl, and in particular, methyl, ethyl, isopropyl and tertiary butyl; and $C_1$ to $C_6$ substituted with phenyl and phenyl substituted with methyl, methoxy or hydroxy, with benzo-1,3-dioxanyl, or with octahydrodimethyldioxopurinyl, and in particular, 1-(4-hydroxyphenyl)prop-2-yl, 2-(2-methoxyphenoxy)ethyl, 1-(benzo-1,3-dioxan-5-yl)-prop-2-yl, 1-(benzo-1,3-dioxan-5-yl)but-3-yl and 3-[3-(1,2,3,4,5,6,8,9-octahydro-7,9-dimethyl-2,8-dioxopurinyl)propyl]; or $R^{56}$ and $R^{57}$ together form a $C_4$ to $C_6$ alkylene linking group, and in particular, pentamethylene.

Arylethanolamine enantiomers of formula XXXIX may be prepared from the compounds of formula I by reduction and, optionally, N-alkylation. Accordingly in a still further embodiment the invention provides a process for the preparation of a compound of formula XXXIX which process comprises the reduction of the nitrile group of an $\alpha$ -substituted- $\alpha$ -cyanomethyl alcohol enantiomer of formula I and, optionally, N-alkylation.

In the above process, the nitrile or cyano group may be reduced directly to an amino group and the amino group directly alkylated or reacted with an aldehyde or a ketone to form an imine and the imine either reduced or alkylated as required to give a compound of formula XXXIX in which $R^{57}$ is hydrogen, as shown below wherein $R^{56}L$ is an alkylating agent and $R^{58}R^{59}C=O$ is an alkyl aldehyde or a dialkyl ketone.

Step 1

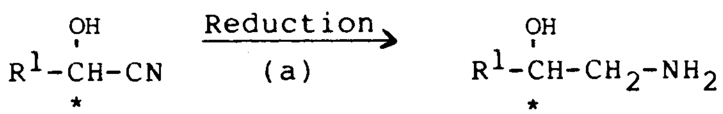

Step 2

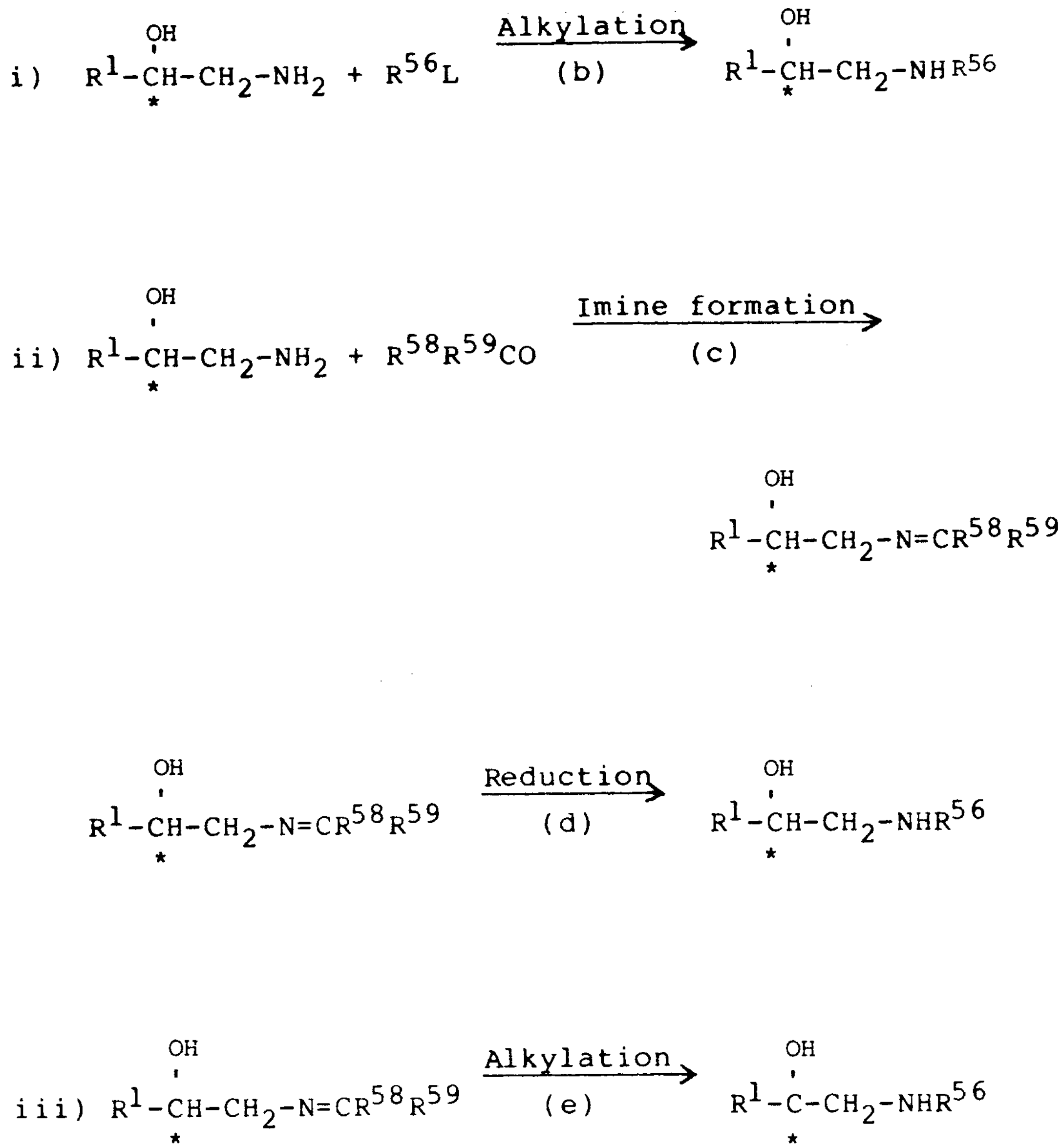


The reduction of the nitrile group to an amino group shown in Step 1 (reaction a) above and the reduction of the imine group to an amino group shown in Step 2 ii) (reaction d) above may be carried out using any of the procedures known in the art for the reduction of nitrile groups to amino groups and which will not racemize the chiral centre. Suitable reducing agents may be selected from sodium borohydrate optionally complexed with cobalt chloride, borane-tetrahydrofuran complex, lithium aluminium hydride, borane - dimethyl sulfide complex, sulfurated sodium borohydride, aluminium hydride in tetrahydrofuran, noble metal catalyst such as platinum or palladium optionally supported on carbon and Raney nickel.

The alkylation of the amino group shown in Step 2 i) (reaction b) may be carried out using any of the procedures known in the art for the N-alkylation of amines and which will not racemize the chiral centre. Suitable alkylating agents may be selected from compounds of formula $R^{56}L$ in which L is a leaving group such as, for example, chloro, bromo or iodo.

The formation of the imine shown in Step 2 ii) (reaction c) may be carried out using any of the procedures known in the art for the condensation of aldehydes or ketones with primary amines to form imines and which will not racemize the chiral centre. It will be recognized by those skilled in the art that the imine formation shown in Step 2 ii) (reaction c) and reduction shown in Step 2 ii) (reaction d) may be

combined in a one step reductive alkylation process.

The alkylation of the imine shown in Step 2 iii) (reaction e) may be carried out using any of the procedures known in the art for the alkylation of imines and which will not racemize the chiral centre. Suitable alkylating agents may be chosen from alkyl Grignard reagents, alkyl cuprates and metal alkyls such as lithium alkyls, sodium alkyls and potassium alkyls.

In an alternative to reactions described above, the above process may be effected by reacting the nitrile or cyano group with an alcohol under Ritter reaction conditions known in the art to give an N-alkyl amide. The N-alkyl amide may then be reduced directly or converted to a thioamide and the thioamide to give a compound of formula XXXIX in which $R^{57}$ is hydrogen, as shown below.

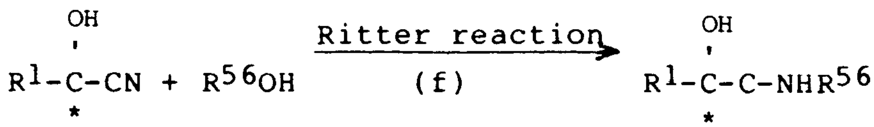


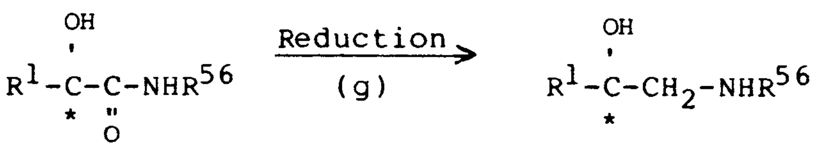


Specific examples of arylethanolamine enantiomers of formula XXXIX which may be prepared from $\alpha$-substituted-$\alpha$-cyanomethyl alcohol enantioners of formula I prepared according to the process of the present invention include those compounds illustrated in Table 5 below.

TABLE 5

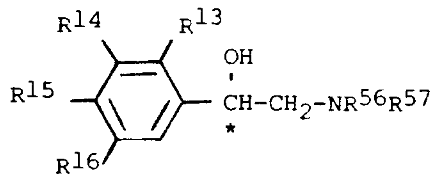

| Substituents | | | | | |
|---|---|---|---|---|---|
| $R^{13}$ | $R^{14}$ | $R^{15}$ | $R^{16}$ | $R^{56}$ | $R^{57}$ |
| H | HO | H | H | H | H |
| H | HO | H | H | $CH_3$ | H |
| H | HO | H | H | $C_2H_5$ | H |
| H | HO | HO | H | H | H |
| H | HO | HO | H | $CH_3$ | H |
| H | HO | HO | H | $C_2H_5$ | H |
| H | HO | HO | H | $CH(CH_3)_2$ | H |
| H | HO | HO | H | $-CH_2CH_2CH_2CH_2CH_2-$ | |
| H | HO | HO | H | a | H |
| H | HO | H | HO | $-CH(CH_3)_2$ | H |
| H | HO | H | HO | $-C(CH_3)_3$ | H |
| H | HO | H | HO | b | H |
| H | HO | H | HO | c | H |
| H | $HOCH_2$ | HO | H | $-C(CH_3)_3$ | H |
| H | $CH_3SO_2NH$ | HO | H | $-CH(CH_3)_2$ | H |
| H | H | HO | H | H | H |
| H | H | HO | H | $CH_3$ | H |
| H | H | HO | H | $-(CH_2)_3CH_3$ | H |
| $H_2NCONH$ | HO | H | H | $-C(CH_3)_3$ | H |
| H | Cl | $H_2N$ | Cl | $-C(CH_3)_3$ | H |
| Cl | H | H | H | $-CH(CH_3)_2$ | H |
| H | $CH_3O$ | HO | $CH_3O$ | $-CH_3$ | H |
| H | $H_2NSO_2$ | $H_3C$ | H | d | H |
| H | H | $NO_2$ | H | $-CH(CH_3)_2$ | H |
| H | $-CH=CH-CH=CH-$ | | H | $-CH(CH_3)_2$ | H |

Code:

a -

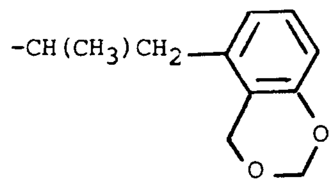

b -

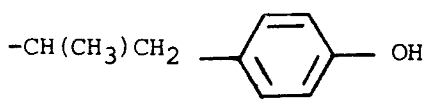

c -

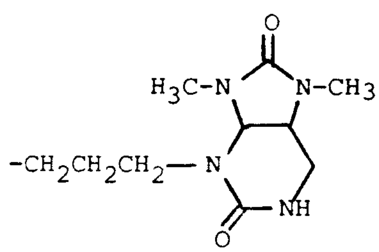

d -

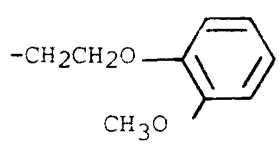

Certain of the arylethanolamine enantiomers of formula XXXIX are believed to be novel compounds per se. Accordingly in further aspects the invention provides novel enantiomers of formula XXXIX prepared from enantiomers of formula I prepared according to the process of the present invention.

The invention is now illustrated by the following Examples.

Example 1

Preparation of (R)-3-Benzyl-(R)-6-(4-imidazolylmethyl) 2,5-piperazinedione

(i) (R)-phenylalanine (9.24 g; 0.056 mol) was dissolved in aqueous sodium hydroxide (28 $cm^3$ of 2 M) and the solution was cooled to °C. Benzylchloroformate (8.8 $cm^3$); 0.06 mol) and aqueous sodium hydroxide (15.3 $cm^3$) were simultaneously added dropwise to the stirred solution over a period of 1 hour. The mixture was stirred at room temperature for 2.5 hours and then extracted with diethyl ether (100 $cm^3$). The aqueous layer was separated and the pH adjusted to 2.0 by the dropwise addition of aqueous 2 M sulfuric acid. The acidified aqueous solution was extracted with ethyl acetate (2 x50 $cm^3$) and the combined organic extracts were dried over anhydrous sodium sulfate and the solvent evaporated. The solid residue was recrystallised from an ethyl acetate/hexane solvent mixture to give N-benzyloxycarbonyl-(R)-phenylalanine (14.09 g; 84%) as a crystalline solid mp 86-87° ($[\alpha]_D$ = -60.4°; C = O.00562 g/$cm^3$; $CHCl_3$).

(ii) (R)-Histidine monohydrochloride monohydrate (5 g; 23.8 mmol) was suspended in methanol (150 $cm^3$) and hydrogen chloride gas was bubbled through the stirred suspension over a period of 2 hours. The solution was heated under reflux for a period of one hour and then diethyl ether (150 $cm^3$) was added to the solution. The precipitated solid was collected by filtration, washed with diethyl ether and dried to give (R)-histidine methyl ester dihydrochloride (5.6 g; 97%) as a solid mp 208-210°C ($[\alpha]_D$ = -7°; C = O.0025 g/$cm^3$; $H_2O$.

(iii) A mixture of N-benzyloxycarbonyl-(R)-phenylalanine (2.67 g; 8.96 mmol), dicyclohexylcarbodiimide (1.84 g; 8.93 mmole), 1-hydroxybenzotriazole (1.20 g; 8.9 mmol) and acetonitrile (60 $cm^3$) was stirred at a temperature of 0°C for a period of one hour. Triethylamine (1.8 g; 2.5 $cm^3$; 18 mmole) was added to a suspension of (R)-histidine methyl ester dihydrochloride (2.17 g; 9 mmol) in acetonitrile (50 $cm^3$) and the suspension was stirred for a period of three hours at room temperature. The (R)-histidine methyl ester mixture was added to the N-benzyloxycarbonyl-(R)-phenylalanine mixture and the resultant mixture was stirred at room temperature for a period of four hours. The precipitated solid was filtered off and the filtrate was evaporated to dryness. The residue was dissolved in chloroform (70 $cm^3$) and the solution was washed with saturated aqueous sodium hydrogen carbonate solution (40 $cm^3$) and then with water (40 $cm^3$). The organic layer was dried over anhydrous sodium sulfate and the solvent evaporated. The residue was recrystallized from a dichloromethane/petroleum ether (bp 40-60°C) solvent mixture to give N-benzyloxycarbonyl-(R)-phenylalanyl-(R)-histidine methyl ester (3.0 g; 74%) as a crystalline solid mp 114-116°C ($[\alpha]_D$ = + 12.7° c = 0.0024 g/$cm^3$; $CHCl_3$).

(iv) N-Benzyloxycarbonyl-(R)-phenylalanyl-(R)-histidine methyl ester (2.95 g; 6.55 mmol) was dissolved in methanol (60 $cm^3$), palladium - black catalyst (200 mg) was added and the solution was hydrogenated for a period of 6 hours. The catalyst was removed by filtration and the methanolic filtrate was heated under reflux for a period of 2 days. The solvent was evaporated and the residue was recrystallized from water to give (R)-3-benzyl-(R)-6-(4-imidaz olylmethyl)-2,5-piperazinedione (1.12 g; 60%) as a crystalline solid mp 259-263° (decomp.) ($[\alpha]_D$ = + 74.7°; C = O.0174 g/$cm^3$; $CH_3COOH$).

Example 2

Preparation of (R)-3-Benzyl-(R)-6-(4-imidazolylmethyl)-2,5-piperazinedione

(i) (R)-Phenylalanine (1.65 g; 10 mmol) was dissolved in aqueous sodium hydroxide (5 $cm^3$; 10 mmol of 2 M) and the solution was cooled to 0°C in an ice-bath. Benzyloxycarbonyl chloride (1.9 g; 11 mmol) and aqueous sodium hydroxide (6 $cm^3$; 12 mmol of 2 M) were simultaneously added to the stirred solution over a period of 15 minutes. The mixture was stirred at 0°C for a period of one hour and then at room temperature for a period of 2.5 hours. The mixture was extracted with diethyl ether and the aqueous layer was separated and adjusted to pH 2.0 by the dropwise addition of concenrtrated hydrochloric acid. The acidified aqueous mixture was extracted with chloroform and the organic extract was dried over anhydrous sodium sulfate and the solvent evaporated. The solid residue was recrystallized from a chloroform/petroleum ether solvent mixture to give N-benzyloxycarbonyl-(R)-phenylalanine (2.69 g; 90% as a crystalline solid mp 87-89°C ($[\alpha]_D$ = -5.0 C = O.01428 g/$cm^3$; $C_2H_5OH$).

(ii) A solution of dicyclohexylcarbodiimide (2.1 g; 10 mmol) in ethyl acetate (10 $cm^3$) was added dropwise to a stirred ice-cold mixture of N-benzyloxycarbonyl-(R)-phenylalanine (3.0 g; 10 mmol), 4-nitrophenol (1.7 g; 12 mmol) and ethyl acetate (30 $cm^3$). The mixture was stirred at a temperature of 0°C for a period of 30 minutes and then at room temperature for a period of 2.5 hours. The precipitated solid was filtered off and the filtrate was concentrated to give a crystalline product which was recrystallized from ethanol to give N-benzyloxycarbonyl-(R)-phenylalanine 4-nitrophenyl ester (1.91 g; 45% as a white solid

mp 120-123° C (/ α /$_D$ = + 8.0; c = 0.01627 g/$cm^3$; $CHCl_3$).

(iii) A mixture of (R)-histidine monohydrochloride monohydrate (4.97 g; 24 mmole) and methanol (50 $cm^3$) was stirred and heated under reflux for a period of one hour while dry hydrogen chloride gas was bubbled through the mixture. The mixture was then cooled in ice and the crystalline product was collected by filtration, washed with diethyl ether and dried to give (R)-histidine methyl ester dihydrochloride (5.35 g; 93%) as a solid mp 208° C (decomp) ([ a ]$_D$ = -9.6; C = 0.01114 g/$cm^3$ $H_2O$).

(iv) (R)-Histidine methyl ester dihydrochloride (0.97 g; 4 mmol) was suspended in a mixture of acetonitrile (10 ml) and triethylamine (1.12 ml; 8 mmol) and the mixture was stirred at room temperature for a period of 5 hours. A suspension of N-benzyloxycarbonyl-(R)-phenyl-alanine 4-nitrophenyl ester (1.68 g; 4 mmol) in acetonitrile (10 ml) was added and the mixture was stirred at room temperature for a period of 2 days. The solvent was evaporated and the solid was washed with diethyl ether and then dissolved in chloroform and the chloroform solution was washed twice with aqueous 10% ammonium hydroxide solution and then twice with water and dried over anhydrous sodium sulfate. The solvent was evaporated and the residue was recrystallised from a dichloromethane/petroleum ether solvent mixture to give N-benzyloxycarbonyl-(R)-phenylalanyl-(R)-histidine methyl ester (1.27 g; 71%) as a crystalline solid mp 113-116° C ([ α ]$_D$ = -13.2; C = 0.00624 g/$cm^3$; MeOH).

(v) N-Benzyloxycarbonyl-(R)-phenylalanyl-(R)-histidine methyl ester (1.2 g; 2.7 mmol) was dissolved in methanol (50 $cm^3$), freshly prepared palladium - black catalyst (300 mg) was added and the mixture was hydrogenated for a period of 7 hours. The catalyst was removed by filtration and the filtrate was heated under reflux for a period of 66 hours. The solvent was evaporated and the residue was recrystallized from water and the product dried overnight over phosphorus pentoxide to give (R)-3-benzyl-(R)-6-(4-imidazolylmethyl)-2,5-piperazinedione (0.72 g; 96%) as a crystalline solid mp 276-278° C ([ α ]$_D$ = + 72.4; C = 0.003 g/$cm^3$; $CH_3COOH$).

Examples 3 to 9

The cyclic dipeptide enantiomers described in Table 6 below were prepared following essentially the same procedure as the described in Example 1 or Example 2 above.

TABLE 6

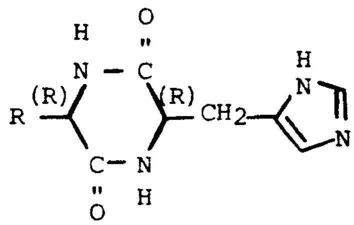

| Example No | R | $[\alpha]_D$ in $CH_3COOH$ (Conc. in $g/cm^3$) | mp °C |
|---|---|---|---|
| 3 | $C_6H_5CH_2$ | +35° (0.0092) | 230-232 (decomp) |
| 4 | $4-HOC_6H_4CH_2$ | +48° (0.0081) | 268-270 |
| 5 | $4-C_6H_5CH_2OC_6H_4CH_2$ | +59° (0.016) | 192-195 |
| 6 | $4-CH_3OC_6H_4CH_2$ | +85° (0.0014) | 238-240 |
| 7* | $3-FC_6H_4CH_2$ | +55° (0.0026) | 270-285 |
| 8 | $C_6H_5$ | +31° (0.008) | 265-270 (decomp) |
| 9 | $CH_3$ | +38° (0.014) | 249-250 |

* (R,S)-3-fluorophenylalanyl-(R)-histidine

Example 10

Preparation of (R)- α -Cyano-3-phenoxybenzyl Alcohol

A mixture of (S)-3-benzyl-(S)-6-(4-imidazolylmethyl)-2,5-piperazinedione (0.06 g; 0.2 mmole) and benzene (4.0 $cm^3$) was cooled under nitrogen in an ice bath. 3-Phenoxybenzaldehyde (2.0 ml; 12 mmole) and then hydrogen cyanide (0.8 $cm^3$; 20 mmole) were rapidly added to the cooled, stirred mixture. The mixture was stirred for 19 hours by which time it had become clear.

The excess hydrogen cyanide and the benzene were removed by evaporation under reduced pressure to give an oil. Diethyl ether (20 $cm^3$) was added to the oil and the precipitated (S)-3-benzyl-(S)-6-(4-imidazolylmethyl)-2,5-piperazinedione was collected by filtration and washed thoroughly with diethyl ether (20 $cm^3$). The combined filtrate and washings were concentrated under reduced pressure to remove the solvent and give a pale yellow oil.

The oil was analysed by proton nuclear magnetic resonance spectroscopy and found to contain 83 mole percent α -cyano-3-phenoxybenzyl alcohol and 17 mole percent unreacted 3-phenoxybenzaldehyde. The optical rotation of the mixture ([ α ]$^{34}$ = + 12.0; 3.54 g/dl; benzene), which, based on the optical rotation of (S)- α -cyano-3-phenoxybenzyl alcohol * ([ α ]D$^{20}$ = 16.5; 0.008 g/cm$^3$; benzene), after correction for the mole fraction of α -cyano-3-phenoxybenzyl alcohol in the product mixture, indicates that (R)- α -cyano-3-phenoxybenzyl alcohol had been formed in 73% enantiomeric excess.

## Example 11

### Preparation of (R)- α -Cyano-3-phenoxybenzyl Alcohol

The procedure described in Example 10 was repeated using a reaction time of 26 hours.

Proton nuclear magnetic resonance spectroscopic analysis of the product showed a 71 mole percent conversion and the optical rotation of the product ([ α ]$_D^{34}$ = + 11.7; 0.0342 g/cm$^3$; benzene) showed, after correction, that (R)- α -cyano-3-phenoxybenzyl alcohol had been formed in 71% enantiomeric excess.

## Example 12

### Preparation of (R)- α -Cyano-3-phenoxybenzyl Alcohol

The (S)-3-benzyl-(S)-6-(4-imidazolylmethyl)-2,5-piperazinedione collected by filtration from the reactions described in Examples 10 and 11 was recrystallized from water and partially dried under vacuum until crusty and granular.

The procedure described in Example 10 was repeated using (S)-3-benzyl-(S)-6-(4-imidazolylmethyl)-2,5-piperazinedione recovered as described above and a reaction time of 16 hours.

Proton nuclear magnetic resonance spectroscopic analysis of the product showed an 86 mole% conversion and the optical rotation of the product ( [ α ]$_D^{34}$ = + 12.0; 0.02966 g/cm$^3$; benzene) showed, after correction, that (R)- α -cyano-3-phenoxybenzyl alcohol had been formed in 75% enantiomeric excess.

## Example 13

### Preparation of (R)- α -Cyano-3-phenoxybenzyl Alcohol

The procedure described in Example 10 was repeated using (S)-3-benzyl-(S)-6-(4-imidazolylmethyl)-2,5-piperazinedione recovered as described in Example 12, toluene as solvent, a reaction temperature of -10 °C and a reaction time of 17.5 hours.

Proton nuclear magnetic resonance spectroscopic analysis of the product showed an 88 mole % conversion and the optical rotation of the product ([ α ]$_D^{34}$ = + 16.4; 3.593 g/dl; benzene) showed, after correction, that (R)- α -cyano-3-phenoxybenzyl alcohol had been formed in 99% enantiomeric excess.

## Example 14

### Preparation of (S)- α -Cyano-3-phenoxybenzyl Alcohol

A mixture of freshly prepared (R)-3-benzyl-(R)-6-(4-imidazolylmethyl)-2,5-piperazinedione (0.64 g; 2.1 mmol) and toluene (40 ml) was cooled under nitrogen to a temperature of -10 °C. 3-Phenoxybenzaldehyde (2.1 cm$^3$; 126 mmol) and then hydrogen cyanide (6 cm$^3$; 150 mmol) were rapidly added to the cooled, stirred mixture. The mixture was stirred for 18.5 hours at -10 °C by which time it had become clear.

The excess hydrogen cyanide and the benzene were removed by evaporation under reduced pressure to give an oil. Diethyl ether (50 cm$^3$) was added to the oil and the precipitated (R)-3-benzyl-(R)-6-(4-imidazolylmethyl)-2,5-piperazinedione was collected by filtration and washed thoroughly with diethyl ether (50 cm$^3$). The combined filtrate and washings were concentrated under reduced pressure to remove the solvent and give a pale yellow oil.

The oil was analysed by proton nuclear magnetic resonance spectroscopy and found to contain 93 mole percent α -cyano-3-phenoxybenzyl alcohol and 7 mole percent unreacted 3-phenoxybenzaldehyde. The optical rotation of the mixture ([ α ]$_D^{34}$ = 11.6 °; 6.921 kg/m$^3$; benzene), which, based on the optical

* Reported in United Kingdom Patent Application No CB-A-2 013 670

rotation of (S)- α -cyano-3-phenoxybenzyl alcohol * ([ α $]_D^{20}$ = -16.5; 0.008 g/$cm^3$; benzene), after correction for the mole fraction of α -cyano-3-phenoxybenzyl alcohol in the product mixture, indicated that (S)- α -cyano-3-phenoxybenzyl alcohol had been formed in 70% enantiomeric excess.

## Examples 15 to 17

The procedure described in Example 14 above was repeated using recovered and recrystallised (see Example 12) (R)-3-benzyl-(R)-6-(4-imidazolylmethyl)-2,5-piperazinedione and the results are reported in Table 7 below.

TABLE 7

| Example No | Reaction Time hours | Yield % | [ α $]_D$ (conc. in g/$cm^3$ solvent) | Enantiomeric Excess |
|---|---|---|---|---|
| 15 | 19 | 84 | -16.5 (0.0354/ $C_6H_6$) | 100[a] |
| 16 | 19 | 84 | -17.9 (0.0362/$C_6H_6$) | 108[a]<br>90[b] |

TABLE 7 - continued

| Example No | Reaction Time hours | Yield % | [ α $]_D$ (conc. in g/$cm^3$ solvent) | Enantiomeric Excess |
|---|---|---|---|---|
| 16 | 19 | 84 | -23.4 (0.0159/$CCl_4$) | 89[c] |
| 17 | 16 | 89 | -26.1 (0.0192/$CCl_4$) | 99[b]<br>89[c] |

Code:

a - Enantiomeric excess based on the optical rotation reported in United Kingdom Patent Application CB-A-2 013 670.

b - Enantiomer excess based on the optical rotation reported in Tetrahedron Letters (1984), 25, 591.

c - Calculated by converting the product alcohol into a pair of diastereo-isomers by esterification with an optically active acid of known optical purity, measuring the proton nuclear magnetic resonance spectrum of the product and integrating the benzylic proton (ie the proton at the chiral centre) which has a different chemical shift for the two different diastereo-isomers.

## Example 18

Preparation of (S)- α -Cyano-3-phenoxybenzyl (IR/S-cis-3-(Z-2-chloro-3,3,3-trifluoroprop-1-enyl)-2,2-dimethylcyclopropanecarboxylate (IR/S, 2R/S; 1 α *S-cyhalothrin)

A solution of dicyclohexylcarbodiimide (0.13 g; 0.6 mmole) in dichloromethane (2 $cm^3$) was added to a

* Reported in United Kingdom Patent Application No CB-A-2 013 670

stirred solution of (IR/S)-cis-3-(Z-2-chloro-3,3,3-trifluoroprop-1-enyl)-2,2-dimethylcyclopropane-carboxylic acid (0.145 g; 0.6 mmole) and (S)- α -cyano-3-phenoxybenzyl alcohol (0.12 g; 0.5 mmole) in dry dichloromethane (3 $cm^3$). The mixture was stirred overnight at room temperature, the dicyclohexylurea was filtered off and the filtrate was concentrated under reduced pressure. The residue was flash chromatographed over silica gel (eluant benzene) and the first 40 $cm^3$ of eluant was collected and the solvent removed by crystallization under reduced pressure to give IR/S, 3R/S; 1 α *S-cyhalothrin 0.24 g as a golden oil. Optical rotation and/or proton nuclear magnetic resonance data is reported in Table 8 below.

Examples 19 to 24

The following pyrethroids were prepared from the corresponding pyrethroid acid and (S)- α cyano-3-phenoxybenzyl alcohol following essentially the same procedure as that described in Example 18.

Example 19 - (S)- α -cyano-3-phenoxybenzyl (1R)-cis-3-(z-2-chloro-3,3,3-trifluoroprop-1-enyl)-2,2-dimethylcarboxylate (1R, 3R/S; 1 α *S-cyhalothrin).

Example 20 - (S)- α -cyano-3-phenoxybenzyl (1R)-cis-3-(2,2-dibromoethenyl)-2,2-dimethylcyclopropanecarboxylate (deltamethrin).

Example 21 - (S)- α -cyano-3-phenoxybenzyl (1R/S)-cis-3-(2,20dichloroethenyl)-2,2-dimethylcyclopropanecarboxylate(1R/S; 1 α *S-cis-cypermethrin).

Example 22 - (S)- α -cyano-3-phenoxybenzyl (1R/S)-trans-3-(2,2-dichloroethenyl)-2,2-dimethylcyclopropanecarboxylate (1R/S; 1 α *S-trans-cypermethrin).

Example 23 - (S)- α -cyano-3-phenoxybenzyl (1R/S)-cis/trans--3-[E/Z-2-chloro-2-(4-chlorophenyl)ethenyl]-2,2-dimethylcyclopropanecarboxylate (1 α *S-flumethrin).

Example 24 - (S)- α -cyano-3-phenoxybenzyl (2R/S)-2-(4-chlorophenyl)-3-methylbutanoate (2R/S; 1 α *S-fenvalerate).

Optical rotation and/or proton nuclear magnetic resonance data is reported in Table 8 below.

TABLLE 8

Physical Data on Synthetic Pyrethroids Prepared from (S)- α -Cyano-3-phenoxybenzyl Alcohol

| Example No | Appearance Melting Point °C | [ α ]$_D$ in $CHCl_3$ (conc in g/cm$^3$) | Chemical Shift (ppm in $CDCl_3$) |
|---|---|---|---|
| 18 | Oil | +10.1 (0.0093) | 1.21-1.33 (6H,m); 1.98-2.25 (2H,m); 6.32, 6.38 (1H, 2xs)*; 6.84 (1H,d, J=9.1Hz); 6.98-7.48 (9H,m) |
| 19 | 55-56 | +35.7 (0.006) | 1.21 (3H,s); 1.29 (3H,s); 1.98-2.28 (2H,m); 6.38 (1H,s); 6.83 (1H,d,J= 8.8Hz); 6.97-7.45 (9H,m) |
| 20 | 100-101 | +16.9 (0.0066) | 1.19 (3H,s); 1.24 (3H,s); 1.84-2.16 (2H,m); 6.37 (1H,s); 6.69 (1H,d,J= 8Hz); 6.97-7.46 (9H,m) |
| | | +18.7 (0.0064) | 1.21 (3H,d); 1.29 (3H,s); 1.84-2.34 (2H,m); 6.32, 6.36 (1H, 2xs)*; 6.17 (1H, d of d, J=8.5, 1.2 Hz); 6.98-7.45 (9H,m) |

TABLE 8 - contined

| | | | |
|---|---|---|---|
| 22 | Oil | +4.8 (0.0066) | 1.18, 1.22, 1.23, 1.33, (6H, 4xs); 1.65 (1H, d of d); 2.30 (1H,m); 6.37, 6.39 (1H,2xs)*; 5.60(1H,d of d, J=8.2, 2.0Hz); 6.98-7.56 (9H, m) |
| 23 | Oil | +3.0 (0.007) | 1.13-1.39 (6H,m); 1.56-2.59 (2H,m); 6.34-6.60 (1H,m); 5.66-5.87 (1H, m); 6.97-7.40 (13H,m) |
| 24 | Oil | -1.4 (0.0051) | 0.71 (3H,d of d); 1.00 (3H,m); 2.34 (1H,m); 3.22 (2H,d, J=10.2Hz); 6.3,6.34 (1H,2xs)*, 6.97-7.74 (13H,m). |

* Multiplicity due to presence of diastereo-isomers.

Example 25

Preparation of (R)- α -Cyano- α (2-naphthyl)methyl Alcohol

A mixture of (S)-3-benzyl-(S)-6-(4-imidazolylmethyl)-2,5-piperazinedione (0.3 g; 0.1 mmole) and toluene (5 $cm^3$) was cooled under nitrogen to a temperature of -10 °C. 2-Naphthaldehyde (0.80 g; 5 mmole) and then hydrogen cyanide (1.0 $cm^3$; 25 mmole) were rapidly added and the mixture was stirred at a temperature of -10 °C for a period of 16 hours. Diethyl ether (25 $cm^3$) was added to the mixture to dissolve the cream coloured solid which had precipitated and to precipitate the dipeptide catalyst. The catalyst was removed by filtration and the filtrate was concentrated under reduced pressure to give (R)- α -cyano- α -(2-naphthyl)methyl alcohol as a cream solid; mp (crude) 103-107 °C; $[\alpha]_D$ = + 16.0 at a concentration of 0.00844 g/$cm^3$ in $CHCl_3$.

Examples 26 to 37

The following arylaldehydes were reacted with hydrogen cyanide either in the presence of (S)-3-benzyl-(S)-6-(4-imidazolylmethyl)-2,5-piperazinedione (code S,S) or (R)-3-benzyl-(R)-6-(4-imidazolylmethyl)-2,5-piperazinedione (code R,R) to produce the corresponding enantiomeric or enantiomer enriched α -aryl- α -cyanomethyl alcohol following essentially the same procedure as that described in Example 25.

TABLE 9

| Example No | Aldehyde | Catalyst |
|---|---|---|
| 26 | 3-methoxybenzaldehyde | S,S |
| 27 | 4-methoxybenzaldehyde | S,S |
| 28 | 4-trifluoromethylbenzaldehyde | S,S |
| 29 | 2-methylbenzaldehyde | R,R |
| 30 | 2-naphthaldehyde | R,R |
| 31 | 3-chlorobenzaldehyde | R,R |
| 32 | 3-acetyloxybenzaldehyde | S,S |
| 33 | 3-nitrobenzaldehyde | S,S |
| 34 | 3-benzyloxybenzaldehyde | R,R |
| 35 | 3-hydroxybenzaldehyde | S,S |
| 36 | 4-chlorobenzaldehyde | S,S |
| 37 | 3,4-bis[1-(ethoxy)ethoxy]benzaldehyde | S,S |

The results are reported in Table 10 below in which the Compound No refers to the $\alpha$ -aryl- $\alpha$ -cyanomethyl alcohols listed in Table 2.

TABLE 10

| Example No | Product Compound No | Reaction Time Hours | Reaction Temp °C | Yield % | [ α ]$_D$ (conc in g/$cm^3$) |
|---|---|---|---|---|---|
| 26 | 21 | 16.5 | -10 | 98 | +25.5 (0.0241)[a] |
| 27 | 22 | 26 | -10 | 75 | +25.9 (0.0271)[b] |
| 28 | 23 | 16 | -10 | 100 | +6.7 (0.0337)[a] |
| 29 | 35 | 19 | -10 | 72 | -12.7 (0.0157)[a] |
| 30 | 24 | 16 | -10 | 100 | -23.5 (0.0048)[a] |
| 31 | 25 | 21 | -10 | 75 | -17.7 (0.034)[a] |
| 32 | 26 | 17 | -10 | 90 | +19.8[c] (0.020)[a] |
| 33 | 27 | 17 | -10 | 95 | +7.1 (0.024)[a] |
| 34 | 29 | 21 | -10 | 64 | -7.2 (0.0124)[b] |
| 35 | 9 | 16[d] | -10 | 50 | +12.9 (0.0174)[e] |
| 36 | 30 | 17 | -10 | 96 | +15.9 (0.01545)[e] |
| 37 | 33 | 17 | -10 | 33 | +6.1 (0.01345)[a] |

Code:

a - [ α ]$_D$ measured in $CHCl_3$

b - [ α ]$_D$ measured in $C_6H_6$

c - Enantiomeric excess 74% (determined as described in

Code c of Table 7.

d - Reaction run in a solvent mixture of 7 parts toluene to 3 parts tetrahydrofuran.

e - [ α ]$_D$ measured in $CH_3OH$

Example 38

Preparation of (R)-2-Amino-1-(2-naphthyl)ethanol

A solution of (R)- α -cyano- α -(2-naphthyl)methyl alcohol (0.37 g; 2 mmole; prepared as described in Example 25) in anhydrous diethyl ether (3 $cm^3$) was added dropwise to a stirred suspension of lithium aluminium hydride (0.17 g; 4.5 mmole) in anhydrous diethyl ether (10 $cm^3$). The mixture was heated under reflux for a period of 2 hours then cooled in an ice bath and water (1 $cm^3$), 10% aqueous sodium hydroxide (2 $cm^3$) and further water (2 $cm^3$) were cautiously added. The organic layer was separated and the inorganic residue was washed several times with diethyl ether. The organic layer and etherial washings were combined, washed with water, dried over anhydrous sodium sulfate and the solvent was removed by evaporation under reduced pressure to give (R)-2-amino-1-(2-naphthyl)ethanol as a white solid (0.33 g; 87%); mp (crude) 115-118 °C; [ α ]$_D$ (crude) = -23.0 ° (C. = 0.00322 g/$cm^3$ in EtOH).

Example 39

Preparation of (R)-2-(N-Isopropylamino)-1-(2-naphthyl)ethanol

A mixture of (R)-2-amino-1-(2-naphthyl)ethanol (0.08 g; 0.4 mmole; prepared as described in Example 38 above), acetone (10 $cm^3$) and ethanol (10 $cm^3$) was hydrogenated at room temperature and pressure for a period of 16 hours in the presence of platinum oxide. The platinum oxide catalyst was removed by filtration and the filtrate evaporated under reduced pressure to give a white solid. The residue was treated with aqueous 1M sodium hydroxide solution and the resulting mixture was extracted with diethyl ether. The organic extract was dried over anhydrous sodium sulfate and the solvent was evaporated under reduced pressure to give a white waxy solid. The solid was recrystallized from ethyl acetate to give (R)-2-(N-isopropylamino)-1-(2-naphthyl)ethanol as a white powder (0.09 g; 92%); mp 93-95 °C; [ α ]$_D$ = -45.0 ° (C. = 0.00302 g/$cm^3$ in $CHCl_3$).

**Claims**

**1.** A process for the preparation of an α -substituted- α -cyanomethyl alcohol enantiomer of formula I

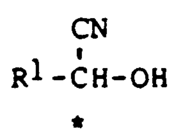

I

which comprises reacting an aldehyde of formula II

$R^1$-CHO II

with hydrogen cyanide in the presence of a cyclic dipeptide enantiomer, and wherein said reaction is carried out at a temperature below ambient temperature
wherein $R^1$ is selected from:

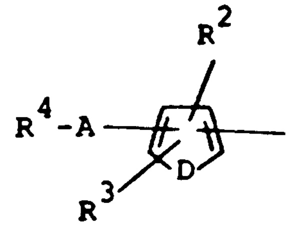

III

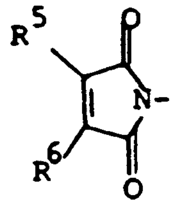

IV

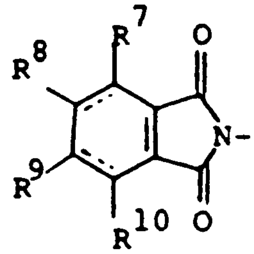

V

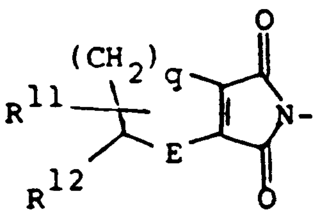

VI

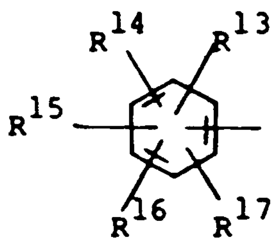

VII

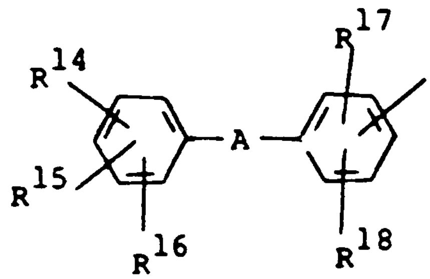

VIII

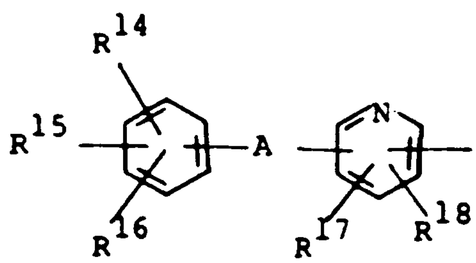

IX

$R^{20}R^{21}C=C(R^{19})-$

X

$R^{22}CH_2-C=C-$

XI

wherein:

$R^2$ and $R^3$ are independently selected from hydrogen, halogen, $C_1$ to $C_6$ alkyl and $C_1$ to $C_6$ haloalkyl, or $R^2$ and $R^3$ jointly form a trimethylene or tetramethylene bridging group;

A is selected from oxygen, sulfur, -CO- and $-CH_2-$;

$R^4$ is selected from hydrogen, $C_1$ to $C_6$ alkyl, $C_2$ to $C_6$ alkenyl, $C_2$ to $C_6$ alkynyl, phenyl, furyl, thienyl and the groups phenyl, furyl and thienyl wherein each group is substituted by halogen, $C_1$ to $C_6$ alkyl, $C_1$ to $C_6$ alkoxy or $C_2$ to $C_6$ alkenyl;

D is selected from oxygen and sulfur;

$R^5$ and $R^6$ are independently selected from $C_1$ to $C_6$ alkyl;

$R^7$, $R^8$, $R^9$ and $R^{10}$ are independently selected from hydrogen, halogen, and $C_1$ to $C_6$ alkyl;

$R^{11}$ and $R^{12}$ are independently selected from hydrogen, $C_1$ to $C_6$ alkyl and halogen, or $R^{11}$ and $R^{12}$ jointly form a methylenedioxy bridging group;

E is selected from oxygen, sulfur and $-CH_2-$;
q is an integer selected from 1 and 2;
$R^{13}$, $R^{14}$, $R^{15}$, $R^{16}$, $R^{17}$ and $R^{18}$ are independently selected from: hydrogen; halogen; nitro; $C_1$ to $C_6$ alkyl; $C_1$ to $C_6$ haloalkyl; $C_1$ to $C_6$ hydroxyalkyl; $C_2$ to $C_6$ alkenyl; $C_2$ to $C_6$ alkynyl; $C_1$ to $C_6$ alkoxy; $C_1$ to $C_6$ alkylthio; ($C_1$ to $C_6$ alkoxy) carbonyl; benzyloxy, substituted benzyloxy; acyloxy; hydroxy; tri($C_1$ to $C_6$ alkyl)silyloxy; ($C_1$ to $C_6$ alkoxy) $C_1$ to $C_6$ alkoxy;$C_1$ to $C_6$ alkoxy-$C_1$ to $C_6$ alkoxymethoxy; amino; N-($C_1$ to $C_6$ alkyl)amino; N,N-di($C_1$ to $C_6$ alkyl)amino; N-($C_1$ to $C_6$ alkanoyl)amino; N-($C_1$ to $C_6$ alkylsulfonyl)-amino; N-(benzenesulfonyl)amino; N-(substituted benzene-sulfonyl)amino; ureido; N-[tri($C_1$ to $C_6$ alkyl)silyl]amino; sulfamoyl; N-($C_1$ to $C_6$ alkyl)sulfamoyl; N,N-di($C_1$ to $C_6$ alkyl)-sulfamoyl; carbamoyl; N-($C_1$ to $C_6$ alkyl)carbamoyl; N,N-di($C_1$ to $C_6$ alkyl)carbamoyl; $C_1$ to $C_6$ alkylsulfinyl; $C_1$ to $C_6$ alkylsulfonyl; thienyloxy; thenyl; furylmethyl; or two adjacent substituents are selected from the linking group buta-1,3-dienylene; and the groups $R^{24}R^{25}C=C(R^{23})O-$ and $R^{24}R^{25}C=C(R^{23})-$ in which $R^{23}$ is selected from hydrogen and $C_1$ to $C_6$ alkyl and $R^{24}$ and $R^{25}$ are independently selected from hydrogen, halogen, $C_1$ to $C_6$ alkyl and $C_1$ to $C_6$ haloalkyl;
$R^{19}$ is selected from hydrogen, chlorine and $C_1$ to $C_6$ alkyl;
$R^{20}$ and $R^{21}$ are independently selected from hydrogen, fluorine, chlorine, bromine, $C_1$ to $C_6$ alkyl, $C_2$ to $C_6$ alkenyl, $C_2$ to $C_6$ alkynyl, phenyl, benzyl, furylmethyl and thienylmethyl; and
$R^{22}$ is selected from phenyl, phenoxy and the groups phenyl and phenoxy wherein each group is substituted by halogen, nitro, cyano, $C_1$ to $C_6$ alkyl, $C_1$ to $C_6$ halo-alkyl or $C_1$ to $C_6$ alkoxy;
and wherein said cyclic dipeptide enantiomer comprises the residues of two amino acids selected from alanine, cysteine, histidine, homoserine, isoleucine, leucine, lysine, methionine, norleucine, norvaline, ornithine, phenylalanine, serine, thyronine, tryptophan, tyrosine, valine, and the N-alkyl, N-alkenyl and N-acyl derivatives thereof.

**2.** A process according to claim 1 wherein in the groups of formulae III, IV, V, VI, VII, VIII, IX, X and XI:
$R^2$ and $R^3$ are independently selected from hydrogen, halogen, $C_1$ to $C_6$ alkyl and $C_1$ to $C_6$ haloalkyl, or $R^2$ and $R^3$ jointly form a trimethylene or tetramethylene bridging group;
A is selected from oxygen, sulfur, -CO- and $-CH_2-$;
$R^4$ is selected from hydrogen, $C_1$ to $C_6$ alkyl, $C_2$ to $C_6$ alkenyl, $C_2$ to $C_6$ alkynyl, phenyl, furyl, thienyl and the groups phenyl, furyl and thienyl wherein each group is substituted by halogen, $C_1$ to $C_6$ alkyl, $C_1$ to $C_6$ alkoxy or $C_2$ to $C_6$ alkenyl;
D is selected from oxygen and sulfur;
$R^5$ and $R^6$ are independently selected from $C_1$ to $C_6$ alkyl;
$R^7$, $R^8$, $R^9$, $R^{10}$ are independently selected from hydrogen, halogen, and $C_1$ to $C_6$ alkyl;
$R^{11}$ and $R^{12}$ are independently selected from hydrogen, $C_1$ to $C_6$ alkyl and halogen, or $R^{11}$ and $R^{12}$ jointly form a methylenedioxy bridging group;
E is selected from oxygen, sulfur and $-CH_2-$;
q is an integer selected from 1 and 2;
$R^{13}$ is selected from hydrogen, halogen, $C_1$ to $C_6$ alkyl, $C_2$ to $C_6$ alkenyl, $C_2$ to $C_6$ alkynyl, thienyloxy, thenyl, furylmethyl and the groups $R^{24}R^{25}C=C(R^{23})0-$ and $R^{24}R^{25}C=C(R^{23})-$ in which $R^{23}$ is selected from hydrogen and $C_1$ to $C_6$ alkyl and $R^{24}$ and $R^{25}$ are independently selected from hydrogen, halogen, $C_1$ to $C_6$ alkyl and $C_1$ to $C_6$ haloalkyl;
$R^{14}$, $R^{15}$, $R^{16}$, $R^{17}$ and $R^{18}$ are independently selected from hydrogen, $C_1$ to $C_6$ alkyl, halogen and $C_2$ to $C_6$ alkenyl;
$R^{19}$ is selected from hydrogen, chlorine and $C_1$ to $C_6$ alkyl;
$R^{20}$ and $R^{21}$ are independently selected from hydrogen, fluorine, chlorine, bromine, $C_1$ to $C_6$ alkyl, $C_2$ to $C_6$ alkenyl, $C_2$ to $C_6$ alkynyl, phenyl, benzyl, furylmethyl and thienylmethyl; and
$R^{22}$ is selected from phenyl, phenoxy and the groups phenyl and phenoxy wherein each group is substituted by halogen, nitro, cyano, $C_1$ to $C_6$ alkyl, $C_1$ to $C_6$ halo-alkyl or $C_1$ to $C_6$ alkoxy.

**3.** A process according to claim 1 or claim 2 wherein $R^1$ is selected from groups of the formulae

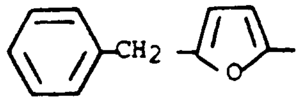

IIIa

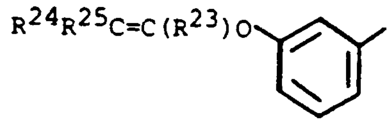

IIIb

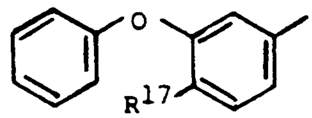

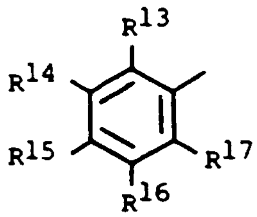

VIIb

VIIIa

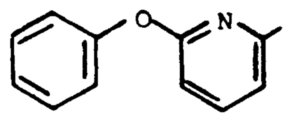

IXa

wherein:
in formula VIIIa, $R^{17}$ is selected from hydrogen and halogen;
in formula VIIb, $R^{13}$, $R^{14}$, $R^{15}$, $R^{16}$ and $R^{17}$ are independently selected from hydrogen, halogen, nitro, hydroxy, $C_1$ to $C_6$ alkyl, $C_1$ to $C_6$ haloalkyl, $C_1$ to $C_6$ hydroxyalkyl, $C_1$ to $C_6$ alkoxy, ($C_1$ to $C_6$ alkoxy)$C_1$ to $C_6$ alkoxy, amino, N-($C_2$ to $C_6$ alkanoyl)amino, N-($C_1$ to $C_6$ alkylsulfonyl) amino, sulfamoyl, ureido, benzyloxy, benzoyloxy, or two adjacent substituents are selected from the linking group buta-1,3-dienylene; and in formula VIIa, $R^{23}$ is hydrogen and $R^{24}$ and $R^{25}$ are independently selected from hydrogen and halogen.

**4.** A process according to claim 3 wherein: in formula VIIIa, $R^{17}$ is selected from hydrogen and fluorine;
in formula VIIb, $R^{13}$, $R^{14}$, $R^{15}$, $R^{16}$ and $R^{17}$ are independently selected from hydrogen, halogen, methyl, trifluoromethyl, hydroxymethyl, methoxy, 1-(ethoxy)ethoxy, nitro, amino, acetamido, methanesulfonylamino, sulfamoyl, ureido, benzyloxy, benzoyloxy, or two adjacent substituents are selected from the linking group buta-1,3-dienylene;
in formula VIIa, $R^{23}$ is hydrogen and $R^{24}$ and $R^{25}$ are independently selected from hydrogen and halogen.

**5.** A process according to any one of claims 1 to 4 inclusive for the preparation of a compound selected from (S)- $\alpha$ -cyano-3-phenoxybenzyl alcohol and (R)-$\alpha$ -cyano-3-phenoxybenzyl alcohol.

**6.** A process according to any one of claims 1, 3 and 4 inclusive for the preparation of a compound of formula I wherein $R^1$ is a group of formula VIIb

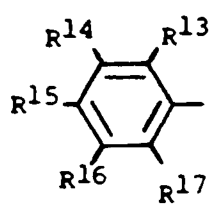

VIIb

wherein;
from one to three of the substituents $R^{13}$, $R^{14}$, $R^{15}$, $R^{16}$ and $R^{17}$ are selected from methoxy, methyl, halogen, hydroxy, nitro, trifluoromethyl, acetyloxy and benzyloxy, or two adjacent substituents are selected from the linking group buta-1,3-dienylene; and
the remaining substituents are hydrogen.

**7.** A process according to anyone of claims 1 to 6 inclusive wherein said cyclic dipeptide enantiomer comprises the residues of two amino acids one of which is selected from histidine and tryptophan and derivatives thereof and the other is selected from phenylalanine, thyronine, tyrosine, tryptophan and histidine and the N-alkyl, N-alkenyl and N-acyl derivatives thereof.

**8.** A process according to any one of claims 1 to 7 inclusive wherein said cyclic dipeptide enantiomer is selected from (R)-3-benzyl-(R)-6-(4-imidazolylmethyl)-2,5-piperazinedione and (S)-3-benzyl-(S)-6-(4-imidazolylmethyl)-2,5-piperazinedione

**9.** A process according to any one of claims 1 to 8 inclusive wherein said cyclic dipeptide enantiomer comprises from 0.5 to 1.5 moles of water of hydration per mole of cyclic dipeptide enantiomer.

**10.** A process according to any one of claims 1 to 9 inclusive wherein said reaction is carried out in the presence of a solvent in which the aldehyde of formula II and hydrogen cyanide are soluble and which is adsorbed by the catalyst.

**11.** A process according to any on of claims 1 to 10 inclusive wherein said reaction is carried out at a temperature at or below 0 ° C.

**12.** A process according to any one of claims 1 to 11 inclusive wherein said reaction is carried out at a temperature below -5 ° C.

**13.** A process according to any one of claims 1 to 12 inclusive wherein said cyclic dipeptide enantiomer is present in an amount in the range of from $10^{-1}$ to $10^{-6}$ moles per mole of the aldehyde of formula II.

**14.** A process for the preparation of a pyrethroid which process comprises the esterification of an $\alpha$ -substituted- $\alpha$ -cyanomethyl alcohol enantiomer of formula I prepared according to the process of any one of claims 1 to 13 inclusive with a pyrethrin acid or a pyrethroid acid or a derivative thereof.

**15.** A process according to claim 14 wherein said $\alpha$ -substituted- $\alpha$ -cyanomethyl alcohol enantiomer is selected from the enantiomers of:
$\alpha$ -cyano-3-phenoxybenzyl alcohol, $\alpha$ -cyano-4-fluoro-3-phenoxybenzyl alcohol, 3-(2,2-dichlorovinyloxy)-$\alpha$ -cyanobenzyl alcohol, $\alpha$ -cyano-pentafluorobenzyl alcohol, $\alpha$ -cyano- $\alpha$ -(6-phenoxypyrid-2-yl)-methylalcohol and $\alpha$ -cyano- $\alpha$ -(5-benzylfur-2-yl)methylalcohol.

**16.** An $\alpha$ -substituted- $\alpha$ -cyanomethyl alcohol enantiomer of formula I

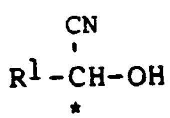

I

wherein $R^1$ is a group of formula VIIb

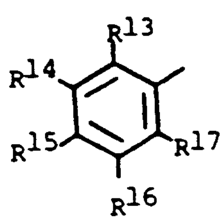

VIIb

wherein:
$R^{13}$, $R^{14}$, $R^{15}$, $R^{16}$ and $R^{17}$ are independently selected from hydrogen, halogen, nitro, hydroxy, $C_1$ to $C_6$ alkyl, $C_1$ to $C_6$ haloalkyl, $C_1$ to $C_6$ hydroxyalkyl, $C_1$ to $C_6$ alkoxy, ($C_1$ to $C_6$ alkoxy)$C_1$to $C_6$ alkoxy, amino, N-($C_2$ to $C_6$ alkanoyl)amino, N-($C_1$ to $C_6$ alkylsulfonyl) amino, sulfamoyl, ureido, benzyloxy, benzoyloxy, or two adjacent substituents are selected from the linking group buta-1,3-dienylene;

with the proviso that at least one or $R^{13}$, $R^{14}$, $R^{15}$, $R^{16}$ and $R^{17}$ is a substituent other than hydrogen.

**Revendications**

1. Procédé de préparation d'un alcool α-cyanométhylique énantiomère substitué en position α, de formule I

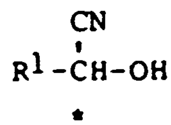

I

qui consiste à faire réagir un aldéhyde de formule II

$R^1$-CHO     II

avec le cyanure d'hydrogène en présence d'un dipeptide cyclique énantiomère, ladite réaction étant conduite à une température inférieure à la température ambiante, formules dans lesquelles $R^1$ est choisi entre

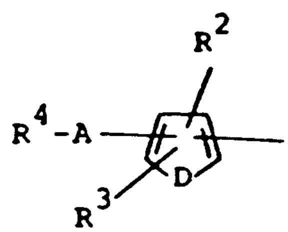

III

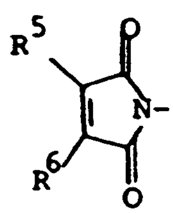

IV

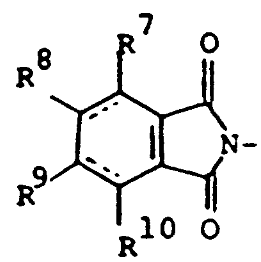

V

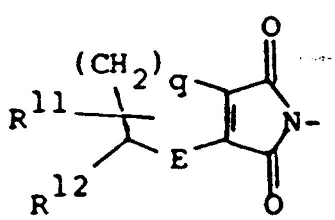

VI

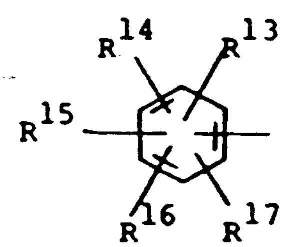

VII

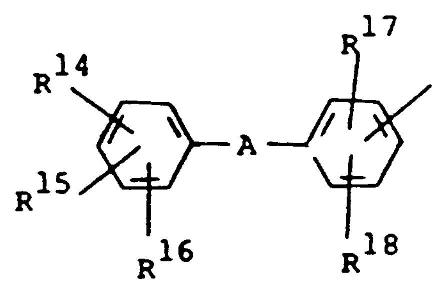

VIII

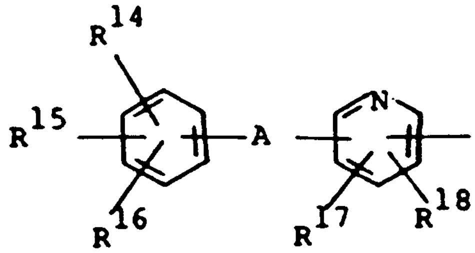

$R^{20}R^{21}C{=}C(R^{19})-$

IX X

$R^{22}CH_2-C{\equiv}C-$

XI

où :

$R^2$ et $R^3$ sont choisis indépendamment entre l'hydrogène, un halogène, un groupe alkyle en $C_1$ à $C_6$ et un groupe halogénalkyle en $C_1$ à $C_6$, ou bien $R^2$ et $R^3$ forment conjointement un groupe triméthylène ou tétraméthylène de pontage ;

A est choisi entre l'oxygène, le soufre, -CO-et $-CH_2-$ ;

$R^4$ est choisi entre l'hydrogène, un groupe alkyle en $C_1$ à $C_6$, alcényle en $C_2$ à $C_6$, alcynyle en $C_2$ à $C_6$, phényle, furyle, thiényle et les groupes phényle, furyle et thiényle dont chacun est substitué par un halogène, un radical alkyle en $C_1$ à $C_6$, alkoxy en $C_1$ à $C_6$ ou alcényle en $C_2$ à $C_6$ ;

D est choisi entre l'oxygène et le soufre ;

$R^5$ et $R^6$ sont choisis, indépendamment, entre des groupes alkyle en $C_1$ à $C_6$ ;

$R^7$, $R^8$, $R^9$ et $R^{10}$ sont choisis indépendamment entre l'hydrogène, un halogène et des groupes alkyle en $C_1$ à $C_6$ ;

$R^{11}$ et $R^{12}$ sont choisis indépendamment entre l'hydrogène, des groupes alkyle en $C_1$ à $C_6$ et un halogène, ou bien $R^{11}$ et $R^{12}$ forment conjointement un groupe méthylènedioxy de pontage ;

E est choisi entre l'oxygène, le soufre et $-CH_2-$ ;

q est un nombre entier choisi entre 1 et 2 ;

$R^{13}$, $R^{14}$, $R^{15}$, $R^{16}$, $R^{17}$ et $R^{18}$ sont choisis indépendamment entre : l'hydrogène ; un halogène ; un groupe nitro ; un groupe alkyle en $C_1$ à $C_6$, halogénalkyle en $C_1$ à $C_6$ ; hydroxyalkyle en $C_1$ à $C_6$ ; alcényle en $C_2$ à $C_6$ ; alcynyle en $C_2$ à $C_6$ ; alkoxy en $C_1$ à $C_6$ ; alkylthio en $C_1$ à $C_6$ ; (alkoxy en $C_1$ à $C_6$)carbonyle ; benzyloxy ; benzyloxy substitué ; acyloxy ; hydroxy ; tri(alkyle en $C_1$ à $C_6$)silyloxy ; (alkoxy en $C_1$ à $C_6$)alkoxy en $C_1$ à $C_6$ ; (alkoxy en $C_1$ à $C_6$)-(alkoxy en $C_1$ à $C_6$)méthoxy ; amino ; N-(alkyle en $C_1$ à $C_6$)amino ; N,N-di(alkyle en $C_1$ à $C_6$)amino ; N-(alcanoyle en $C_1$ à $C_6$)amino ; N-(alkylsulfonyle en $C_1$ à $C_6$)amino ; N-(benzènesulfonyl)amino ; N-(benzènesulfonyle substitué)amino ; uréido ; N-[tri(alkyle en $C_1$ à $C_6$)silyl]amino ; sulfamoyle ; N-(alkyle en $C_1$ à $C_6$)sulfamoyle ; N,N-di-(alkyle en $C_1$ à $C_6$)sulfamoyle ; carbamoyle ; N-(alkyle en $C_1$ à $C_6$)carbamoyle ; N,N-di(alkyle en $C_1$ à $C_6$)carbamoyle ; alkylsulfinyle en $C_1$ à $C_6$ ; alkylsulfonyle en $C_1$ à $C_6$ ; thiényloxy ; thényle ; furylméthyle ; ou bien deux substituants adjacents sont choisis entre le groupe de jonction buta-1,3-diénylène ; et les groupes $R^{24}R^{25}C=C(R^{23})O-$ et $R^{24}R^{25}C=C(R^{23})-$ dans lesquels $R^{23}$ est choisi entre l'hydrogène et des groupes alkyle en $C_1$ à $C_6$ et $R^{24}$ et $R^{25}$ sont choisis indépendamment entre l'hydrogène, un halogène, des groupes alkyle en $C_1$ à $C_6$ et des groupes halogénalkyle en $C_1$ à $C_6$ ;

$R^{19}$ est choisi entre l'hydrogène, le chlore et des groupes alkyle en $C_1$ à $C_6$ ; $R^{20}$ et $R^{21}$ sont choisis indépendamment entre l'hydrogène, le fluor, le chlore, le brome, des groupes alkyle en $C_1$ à $C_6$, alcényle en $C_2$ à $C_6$, alcynyle en $C_2$ à $C_6$, phényle, benzyle, furylméthyle et thiénylméthyle ; et

$R^{22}$ est choisi entre le groupe phényle, phénoxy et les groupes phényle et phénoxy substitués chacun par un halogène, un radical nitro, cyano, alkyle en $C_1$ à $C_6$, halogénalkyle en $C_1$ à $C_6$ ou alkoxy en $C_1$ à $C_6$ ;

et l'énantiomère dipeptidique cyclique comprend les résidus de deux amino-acides choisis entre l'alanine, la cystéine, l'histidine, l'homosérine, l'isoleucine, la leucine, la lysine, la méthionine, la norleucine, la norvaline, l'ornithine, la phénylalanine, la sérine, la thyronine, le tryptophane, la tyrosine,

la valine et leurs dérivés N-alkyliques, N-alcényliques et N-acyliques.

**2.** Procédé suivant la revendication 1, dans les groupes de formules III, IV, V, VI, VII, VIII, IX, X et XI duquel :

$R^2$ et $R^3$ sont choisis indépendamment entre l'hydrogène, un halogène, un groupe alkyle en $C_1$ à $C_6$ et un groupe halogénalkyle en $C_1$ à $C_6$, ou bien $R^2$ et $R^3$ forment conjointement un groupe triméthylène ou tétraméthylène de pontage ;

A est choisi entre l'oxygène, le soufre, -CO-et $-CH_2-$ ;

$R^4$ est choisi entre l'hydrogène, un groupe alkyle en $C_1$ à $C_6$, alcényle en $C_2$ à $C_6$, alcynyle en $C_2$ à $C_6$, phényle, furyle, thiényle et les groupes phényle, furyle et thiényle dont chacun est substitué par un halogène, un radical alkyle en $C_1$ à $C_6$, alkoxy en $C_1$ à $C_6$ ou alcényle en $C_2$ à $C_6$ ;

D est choisi entre l'oxygène et le soufre ;

$R^5$ et $R^6$ sont choisis, indépendamment, entre des groupes alkyle en $C_1$ à $C_6$ ;

$R^7$, $R^8$, $R^9$ et $R^{10}$ sont choisis indépendamment entre l'hydrogène, un halogène et des groupes alkyle en $C_1$ à $C_6$ ;

$R^{11}$ et $R^{12}$ sont choisis indépendamment entre l'hydrogène, des groupes alkyle en $C_1$ à $C_6$ et un halogène, ou bien $R^{11}$ et $R^{12}$ forment conjointement un groupe méthylènedioxy de pontage ;

E est choisi entre l'oxygène, le soufre et $-CH_2-$ ;

q est un nombre entier choisi entre 1 et 2 ;

$R^{13}$ est choisi entre l'hydrogène, un halogène, des groupes alkyle en $C_1$ à $C_6$, alcényle en $C_2$ à $C_6$, alcynyle en $C_2$ à $C_6$, thiényloxy, thényle, furylméthyle et les groupes $R^{24}R^{25}C=C(R^{23})O-$ et $R^{24}R^{25}C=C(R^{23})-$ dans lesquels $R^{23}$ est choisi entre l'hydrogène et des groupes alkyle en $C_1$ à $C_6$ et $R^{24}$ et $R^{25}$ sont choisis indépendamment entre l'hydrogène, un halogène, des groupes alkyle en $C_1$ à $C_6$ et halogénalkyle en $C_1$ à $C_6$ ;

$R^{14}$, $R^{15}$, $R^{16}$, $R^{17}$ et $R^{18}$ sont choisis indépendamment entre l'hydrogène, des groupes alkyle en $C_1$ à $C_6$, un halogène et des groupes alcényle en $C_2$ à $C_6$ ;

$R^{19}$ est choisi entre l'hydrogène, le chlore et des groupes alkyle en $C_1$ à $C_6$ ;

$R^{20}$ et $R^{21}$ sont choisis indépendamment entre l'hydrogène, le fluor, le chlore, le brome, des groupes alkyle en $C_1$ à $C_6$, alcényle en $C_2$ à $C_6$, alcynyle en $C_2$ à $C_6$, phényle, benzyle, furylméthyle et thiénylméthyle ; et

$R^{22}$ est choisi entre les groupes phényle, phénoxy et les groupes phényle et phénoxy substitués chacun par un halogène, un radical nitro, cyano, alkyle en $C_1$ à $C_6$, halogénalkyle en $C_1$ à $C_6$ ou alkoxy en $C_1$ à $C_6$.

**3.** Procédé suivant la revendication 1 ou la revendication 2, dans laquelle $R^1$ est choisi dans les groupes de formules

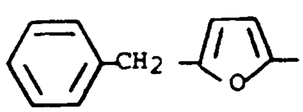

IIIa

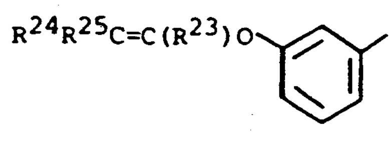

IIIb

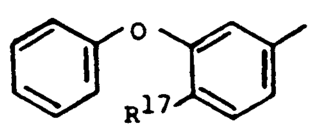

VIIIa

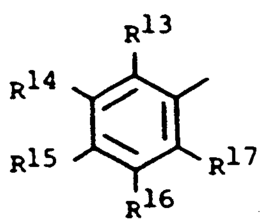

VIIb

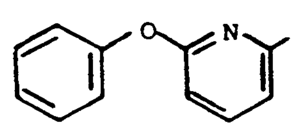

IXa

dans lesquelles :

dans la formule VIIIa, $R^{17}$ est choisi entre l'hydrogène et un halogène ;

dans la formule VIIb, $R^{13}$, $R^{14}$, $R^{15}$, $R^{16}$ et $R^{17}$ sont choisis indépendamment entre l'hydrogène, un halogène, un groupe nitro, hydroxy, alkyle en $C_1$ à $C_6$, halogénalkyle en $C_1$ à $C_6$, hydroxyalkyle en $C_1$ à $C_6$, alkoxy en $C_1$ à $C_6$, (alkoxy en $C_1$ à $C_6$)alkoxy en $C_1$ à $C_6$, amino, N-(alcanoyle en $C_2$ à $C_6$)amino, N-(alkylsulfonyle en $C_1$ à $C_6$)amino, sulfamoyle, uréido, benzyloxy, benzoyloxy ou bien deux substituants adjacents sont choisis comme groupe de jonction buta-1,3-diénylène ; et dans la formule VIIa, $R^{23}$ est l'hydrogène, et $R^{24}$ et $R^{25}$ sont choisis indépendamment entre l'hydrogène et un halogène.

**4.** Procédé suivant la revendication 3, dans lequel :

dans la formule VIIIa, $R^{17}$ est choisi entre l'hydrogène et le fluor ;

dans la formule VIIb, $R^{13}$, $R^{14}$, $R^{15}$, $R^{16}$ et $R^{17}$ sont choisis indépendamment entre l'hydrogène, un halogène, un groupe méthyle, trifluorométhyle, hydroxyméthyle, méthoxy, 1-(éthoxy)éthoxy, nitro, amino, acétamido, méthanesulfonylamino, sulfamoyle, uréido, benzyloxy, benzoyloxy, ou bien deux substituants adjacents sont choisis comme groupe de jonction buta-1,3-diénylène ;

dans la formule VIIa, $R^{23}$ est l'hydrogène et $R^{24}$ et $R^{25}$ sont choisis indépendamment entre l'hydrogène et un halogène.

**5.** Procédé suivant l'une quelconque des revendications 1 à 4 inclus pour la préparation d'un composé choisi entre l'alcool (S)-$\alpha$-cyano-3-phénoxybenzylique et l'alcool (R)- $\alpha$-cyano-3-phénoxybenzylique.

**6.** Procédé suivant l'une quelconque des revendications 1, 3 et 4 inclus pour la préparation d'un composé de formule I dans laquelle $R^1$ est un groupe de formule VIIb

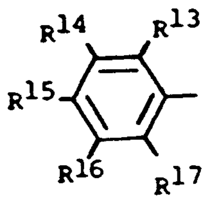

VIIb

dans laquelle :

un à trois des substituants $R^{13}$, $R^{14}$, $R^{15}$, $R^{16}$ et $R^{17}$ sont choisis entre les groupes méthoxy, méthyle, un halogène, les groupes hydroxy, nitro, trifluorométhyle, acétyloxy et benzyloxy, ou bien deux substituants adjacents sont choisis comme groupe buta-1,3-diénylène de jonction ; et

les substituants restants sont de l'hydrogène.

**7.** Procédé suivant l'une quelconque des revendications 1 à 6 inclus, dans lequel l'énantiomère dipeptidique cyclique comprend les résidus de deux aminoacides dont l'un est choisi entre l'histidine et le tryptophane et leurs dérivés et l'autre est choisi entre la phénylalanine, la thyronine, la tyrosine, le tryptophane et l'histidine et leurs dérivés N-alkyliques, N-alcényliques et N-acyliques.

**8.** Procédé suivant l'une quelconque des revendications 1 à 7 inclus, dans lequel l'énantiomère dipeptidique est choisi entre la (R)-3-benzyl-(R)-6-(4-imidazolylméthyl)-2,5-pipérazinedione et la (S)-3-benzyl-(S)-6-(4-imidazolylméthyl)-2,5-pipérazinedione.

**9.** Procédé suivant l'une quelconque des revendications 1 à 8 inclus, dans lequel ledit énantiomère dipeptidique cyclique comprend 0,5 à 1,5 mole d'eau d'hydratation par mole d'énantiomère dipeptidique cyclique.

**10.** Procédé suivant l'une quelconque des revendications 1 à 9 inclus, dans lequel ladite réaction est conduite en présence d'un solvant dans lequel l'aldéhyde de formule (II) et le cyanure d'hydrogène sont solubles et qui est adsorbé par le catalyseur.

**11.** Procédé suivant l'une quelconque des revendications 1 à 10 inclus, dans lequel la réaction est conduite à une température égale ou inférieure à 0 °C.

**12.** Procédé suivant l'une quelconque des revendications 1 à 11 inclus, dans lequel la réaction est conduite à une température inférieure à -5 ° C.

**13.** Procédé suivant l'une quelconque des revendications 1 à 12 inclus, dans lequel l'énantiomère dipeptidique cyclique est présent en une quantité comprise dans la plage de $10^{-1}$ à $10^{-6}$ mole par mole d'aldéhyde de formule II.

**14.** Procédé de préparation d'un pyréthroide, procédé qui consiste à estérifier un alcool $\alpha$-cyanométhylique énantiomère substitué en $\alpha$ de formule I, préparé conformément au procédé suivant l'une quelconque des revendications 1 à 13 inclus, avec un acide de la pyréthrine ou un acide d'un pyréthroide ou un dérivé correspondant.

**15.** Procédé suivant la revendication 14, dans lequel l'alcool $\alpha$-cyanométhylique énantiomère substitué en $\alpha$ est choisi entre les énantiomères de l'alcool $\alpha$-cyano-3-phénoxybenzylique, l'alcool $\alpha$-cyano-4-fluoro-3-phénoxybenzylique, l'alcool 3-(2,2-dichlorovinyloxy)-$\alpha$-cyanobenzylique, l'alcool $\alpha$-cyano-pentafluoro-benzylique, l'alcool $\alpha$-cyano-$\alpha$-(6-phénoxypyrid-2-yl)méthylique et l'alcool $\alpha$-cyano-$\alpha$-(5-benzylfur-2-yl)-méthylique.

**16.** Alcool $\alpha$-cyanométhylique énantiomère substitué en $\alpha$, de formule I

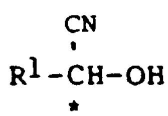

I

dans laquelle $R^1$ est un groupe de formule VIIb

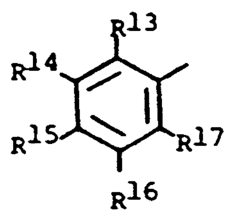

VIIb

dans laquelle :

$R^{13}$, $R^{14}$, $R^{15}$, $R^{16}$ et $R^{17}$ sont choisis indépendamment entre l'hydrogène, un halogène, un groupe nitro, hydroxy, alkyle en $C_1$ à $C_6$, halogénalkyle en $C_1$ à $C_6$, hydroxyalkyle en $C_1$ à $C_6$, alkoxy en $C_1$ à $C_6$, (alkoxy en $C_1$ à $C_6$)alkoxy en $C_1$ à $C_6$, amino, N-(alcanoyle en $C_2$ à $C_6$)amino, N-(alkylsulfonyle en $C_1$ à $C_6$)amino, sulfamoyle, uréido, benzyloxy, benzoyloxy, ou bien deux substituants adjacents sont choisis comme groupe de jonction buta-1,3-diénylène, sous réserve qu'au moins l'un de $R^{13}$, $R^{14}$, $R^{15}$, $R^{16}$ et $R^{17}$ soit un substituant autre que l'hydrogène.

## Patentansprüche

**1.** Verfahren zur Herstellung eines $\alpha$-substituierten $\alpha$-Cyanomethyl-alkohol-Enantiomers der Formel I,

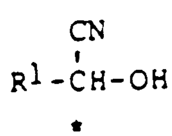

I

bei welchem ein Aldehyd der Formel II

$R^1$-CHO II

mit Cyanwasserstoff in Gegenwart eines cyclischen Dipeptid-Enantiomers umgesetzt wird und bei welchem die Reaktion bei einer Temperatur unterhalb Raumtemperatur ausgeführt wird, wobei $R^1$ ausgewählt ist aus

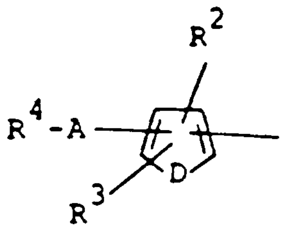
III

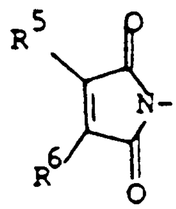
IV

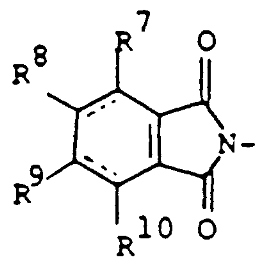
V

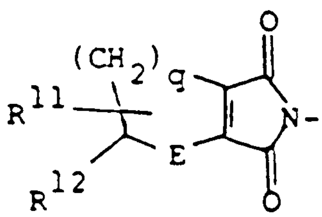
VI

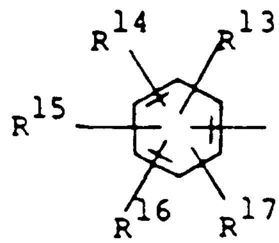
VII

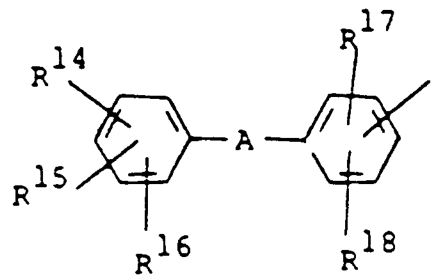
VIII

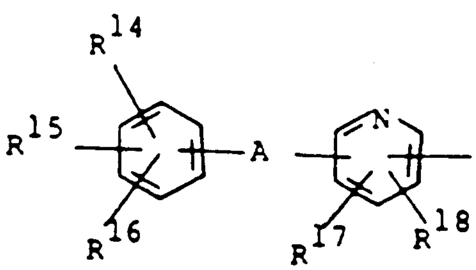
IX

$R^{20}R^{21}C{=}C(R^{19})-$

X

$R^{22}\ CH_2\text{-}C{\equiv}C\text{-}$

XI

worin

$R^2$ und $R^3$ unabhängig ausgewählt sind aus Wasserstoff, Halogen,

$C_{1-6}$-Alkyl und $C_{1-6}$-Halogenoalkyl oder $R^2$ und $R^3$ gemeinsam eine Trimethylen- oder Tetramethylen-Brückengruppe bilden,

A ausgewählt ist aus Sauerstoff, Schwefel, -CO- und $-CH_2-$,

$R^4$ ausgewählt ist aus Wasserstoff, $C_{1-6}$-Alkyl, $C_{2-6}$-Alkenyl,

$C_{2-6}$-Alkinyl, Phenyl, Furyl, Thienyl und den Gruppen Phenyl, Furyl und Thienyl, wobei jede Gruppe substituiert ist durch Halogen, $C_{1-6}$-Alkyl, $C_{1-6}$-Alkoxy oder $C_{2-6}$-Alkenyl,

D ausgwählt ist aus Sauerstoff und Schwefel,

$R^5$ und $R^6$ unabhängig ausgewählt sind aus $C_{1-6}$-Alkyl,
$R^7$, $R^8$, $R^9$ und $R^{10}$ unabhängig ausgewählt sind aus Wasserstoff, Halogen und $C_{1-6}$-Alkyl,
$R^{11}$ und $R^{12}$ unabhängig ausgewählt sind aus Wasserstoff, $C_{1-6}$-Alkyl und Halogen oder $R^{11}$ und $R^{12}$ gemeinsam eine Methylendioxy-Brückengruppe bilden,
E ausgewählt ist aus Sauerstoff, Schwefel und $-CH_2-$,
q für eine aus 1 und 2 ausgewählte Ganzzahl steht,
$R^{13}$, $R^{14}$, $R^{15}$, $R^{16}$, $R^{17}$ und $R^{18}$ unabhängig ausgewählt sind aus Wasserstoff, Halogen, Nitro, $C_{1-6}$-Alkyl, $C_{1-6}$-Halogenoalkyl, $C_{1-6}$-Hydroxyalkyl, $C_{2-6}$-Alkenyl, $C_{2-6}$-Alkinyl, $C_{1-6}$-Alkoxy, $C_{1-6}$-Alkylthio, ($C_{1-6}$-Alkoxy)carbonyl, Benzyloxy, substituiertem Benzyloxy, Acyloxy, Hydroxy, Tri($C_{1-6}$-Alkyl)silyloxy, ($C_{1-6}$-Alkoxy)-$C_{1-6}$-alkoxy, $C_{1-6}$-Alkoxy-$C_{1-6}$-alkoxy-methoxy, Amino, N-($C_{1-6}$-Alkyl)-amino, N,N-Di-($C_{1-6}$-alkyl)amino, N-($C_{1-6}$-Alkanoyl)amino, N-($C_{1-6}$-Alkylsulfonyl)amino, N-(Benzolsulfonyl)amino, N-(substituiertem Benzol-sulfonyl)amino, Ureido, N-[Tri($C_{1-6}$-alkyl)silyl]amino, Sulfamoyl, N-($C_{1-6}$-Alkyl)-sulfamoyl, N,N-Di ($C_{1-6}$-alkyl)sulfamoyl, Carbamoyl, N-($C_{1-6}$-Alkyl)carbamoyl, N,N-Di($C_{1-6}$-alkyl)-carbamoyl, $C_{1-6}$-Alkylsulfinyl, $C_{1-6}$-Alkylsulfonyl, Thienyloxy, Thenyl, Furylmethyl, wobei zwei benachbarte Substituenten auch eine Buta-1,3-dienylen-Brückengruppebilden können, und den Gruppen $R^{24}R^{25}C=C(R^{23})$ O- und $R^{24}R^{25}C=C(R^{23})$-, worin $R^{23}$ ausgewählt ist aus Wasserstoff und $C_{1-6}$-Alkyl und $R^{24}$ und $R^{25}$ unabhängig ausgewählt sind aus Wasserstoff, Halogen, $C_{1-6}$-Alkyl und $C_{1-6}$-Halogenoalkyl,
$R^{19}$ ausgewählt ist aus Wasserstoff, Chlor und $C_{1-6}$-Alkyl,
$R^{20}$ und $R^{21}$ unabhängig ausgewählt sind aus Wasserstoff, Fluor,
Chlor, Brom, $C_{1-6}$-Alkyl, $C_{2-6}$-Alkenyl, $C_{2-6}$-Alkinyl, Phenyl, Benzyl, Furylmethyl und Thienylmethyl und
$R^{22}$ ausgewählt ist aus Phenyl und Phenoxy und den Gruppen Phenyl und Phenoxy, wobei jede Gruppe substituiert ist durch Halogen, Nitro, Cyano, $C_{1-6}$-Alkyl, $C_{1-6}$-Halogenoalkyl oder $C_{1-6}$-Alkoxy, wobei das cyclische Dipeptid-Enantiomer die Reste von zwei Aminosäuren enthält, die ausgewählt sind aus Alanin, Cystein, Histidin, Homoserin, Isoleucin, Leucin, Lysin, Methionin, Norleucin, Norvalin, Ornithin, Phenylalanin, Serin, Thyronin, Tryptophan, Tyrosin, Valin und den N-Alkyl-, N-Alkenyl- und N-Acyl-Derivaten davon.

**2.** Verfahren nach Anspruch 1, bei welchen in den Gruppen der Formeln III, IV, V, VI, VII, VIII, IX, X und XI
$R^2$ und $R^3$ unabhängig ausgewählt sind aus Wasserstoff, Halogen, $C_{1-6}$-Alkyl und $C_{1-6}$-Halogenoalkyl oder $R^2$ und $R^3$ gemeinsam eine Trimethylen- oder Tetramethylen-Brückengruppe bilden,
A ausgewählt ist aus Sauerstoff, Schwefel, -CO- und $-CH_2-$,
$R^4$ ausgewählt ist aus Wasserstoff, $C_{1-6}$-Alkyl, $C_{2-6}$-Alkenyl, $C_{2-6}$-Alkinyl, Phenyl, Furyl, Thienyl und den Gruppen Phenyl, Furyl und Thienyl, wobei jede Gruppe substituiert ist durch Halogen, $C_{1-6}$-Alkyl, $C_{1-6}$-Alkoxy oder $C_{2-6}$-Alkenyl,
D ausgewählt ist aus Sauerstoff und Schwefel,
$R^5$ und $R^6$ unabhängig ausgewählt sind aus $C_{1-6}$-Alkyl,
$R^7$, $R^8$, $R^9$ und $R^{10}$ unabhängig ausgewählt sind aus Wasserstoff, Halogen und $C_{1-6}$-Alkyl,
$R^{11}$ und $R^{12}$ unabhängig ausgewählt sind aus Wasserstoff, $C_{1-6}$-Alkyl und Halogen oder $R^{11}$ und $R^{12}$ gemeinsam eine Methylendioxy-Brückengruppe bilden.
E ausgewählt ist aus Sauerstoff, Schwefel und $-CH_2-$,
q für eine aus 1 und 2 ausgewählte Ganzzahl steht,
$R^{13}$ ausgewählt ist aus Wasserstoff, Halogen, $C_{1-6}$-Alkyl, $C_{2-6}$-Alkenyl, $C_{2-6}$-Alkinyl, Thienyloxy, Thenyl, Furylmethyl und den Gruppen $R^{24}R^{25}C=C(R^{23})O$- und $R^{24}R^{25}C=C(R^{23})$-, worin $R^{23}$ ausgewählt ist aus Wasserstoff und $C_{1-6}$-Alkyl
und $R^{24}$ und $R^{25}$ unabhängig ausgewählt sind aus Wasserstoff und Halogen, $C_{1-6}$-Alkyl und $C_{1-6}$-Halogenoalkyl,
$R^{14}$, $R^{15}$, $R^{16}$, $R^{17}$ und $R^{18}$ unabhängig ausgewählt sind aus Wasserstoff, $C_{1-6}$-Alkyl, Halogen und $C_{2-6}$-Alkenyl,
$R^{19}$ ausgewählt ist aus Wasserstoff, Chlor und $C_{1-6}$-Alkyl,
$R^{20}$ und $R^{21}$ unabhängig ausgewählt sind aus Wasserstoff, Fluor, Chlor, Brom, $C_{1-6}$-Alkyl, $C_{2-6}$-Alkenyl, $C_{2-6}$-Alkinyl, Phenyl, Benzyl, Furylmethyl und Thienylmethyl und
$R^{22}$ ausgewählt ist aus Phenyl, Phenoxy und den Gruppen Phenyl und Phenoxy, wobei jede Gruppe substituiert ist durch Halogen, Nitro, Cyano, $C_{1-6}$- Alkyl, $C_{1-6}$-Halogenoalkyl oder $C_{1-6}$-Alkoxy.

**3.** Verfahren nach Anspruch 1 oder 2, bei welchem $R^1$ ausgewählt ist aus den Gruppen der Formeln

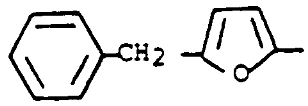

IIIa

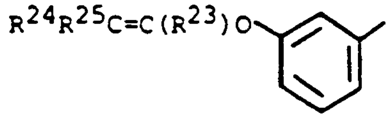

IIIb

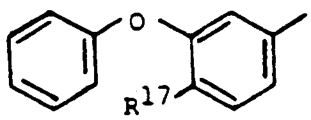

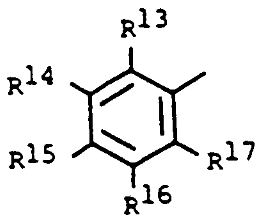

VIIIa

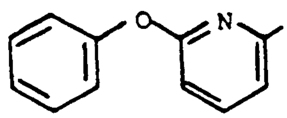

VIIb

IXa

worin
in Formel VIIIa, $R^{17}$ ausgewählt ist aus Wasserstoff und Halogen,
in Formel VIIb $R^{13}$, $R^{14}$, $R^{15}$, $R^{16}$ und $R^{17}$ unabhängig ausgewählt sind aus Wasserstoff, Halogen, Nitro, Hydroxy, $C_{1-6}$-Alkyl, $C_{1-6}$-Halogenoalkyl, $C_{1-6}$-Hydroxyalkyl, $C_{1-6}$-Alkoxy, ($C_{1-6}$-Alkoxy)-$C_{1-6}$-alkoxy, Amino, N-($C_{2-6}$-Alkanoyl)amino, N-($C_{1-6}$-Alkylsulfonyl)amino, Sulfamoyl, Ureido, Benzyloxy und Benzoyloxy, wobei 2 benachbarte Substituenten auch eine Buta-1,3-dienylen-Brückengruppe bilden können, und
in Formel VIIa $R^{23}$ für Wasserstoff steht und $R^{24}$ und $R^{25}$ unabhängig ausgewählt sind aus Wasserstoff und Halogen.

4. Verfahren nach Anspruch 3, bei welchem
in Formel VIIIa $R^{17}$ ausgewählt ist aus Wasserstoff und Fluor,
in Formel VIIb $R^{13}$, $R^{14}$, $R^{15}$, $R^{16}$ und $R^{17}$ unabhängig ausgewählt sind aus Wasserstoff, Halogen, Methyl, Trifluoromethyl, Hydroxymethyl, Methoxy, 1-(Ethoxy)ethoxy, Nitro, Amino, Acetamido, Methansulfonylamino, Sulfamoyl, Ureido, Benzyloxy und Benzoyloxy, wobei zwei benachbarte Substituenten auch eine Buta-1,3-dienylen-Brückengruppe bilden können, und
in Formel VIIa $R^{23}$ für Wasserstoff steht und $R^{24}$ und $R^{25}$ unabhängig ausgewählt sind aus Wasserstoff und Halogen.

5. Verfahren nach einem der Ansprüche 1 bis 4 zur Herstellung einer Verbindung, die ausgewählt ist aus (S)-α-Cyano-3-phenoxybenzyl-alkohol und (R)-α-Cyano-3-phenoxybenzyl-alkohol.

6. Verfahren nach einem der Ansprüche 1, 3 und 4 zur Herstellung einer Verbindung der Formel I, worin $R^1$ für eine Gruppe der Formel VIIb

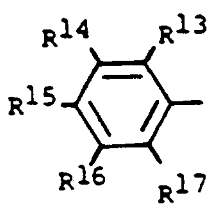

VIIb

steht, worin 1 bis 3 der Substituenten $R^{13}$, $R^{14}$, $R^{15}$, $R^{16}$ und $R^{17}$ ausgewählt sind aus Methoxy, Methyl, Halogen, Hydroxy, Nitro, Trifluoromethyl, Acetyloxy und Benzoyloxy, wobei zwei benachbarte Substituenten auch eine Buta-1,3-dienylen-Brückengruppe bilden können, und die übrigen Substituenten für Wasserstoff stehen.

**7.** Verfahren nach einem der Ansprüche 1 bis 6, bei welchem das cyclische Dipeptid-Enantiomer die Reste von zwei Aminosäuren enthält, von denen eine ausgewählt ist aus Histidin und Tryptophan und Derivaten davon und die andere ausgewählt ist aus Phenylalanin, Thyronin, Tyrosin, Tryptophan und Histidin und den N-Alkyl-, N-Alkenyl- und N-Acyl-Derivaten davon.

**8.** Verfahren nach einem der Ansprüche 1 bis 7, bei welchem das cyclische Dipeptid-Enantiomer ausgewählt ist aus (R)-3-Benzyl-(R)-6-(4-imidazolylmethyl)-2,5-piperazindion und (S)-3-Benzyl-(S)-6-(4-imidazolylmethyl)-2,5-piperazindion.

**9.** Verfahren nach einem der Ansprüche 1 bis 8, bei welchem das cyclische Dipeptid-Enantiomer 0,5 bis 1,5 Mol Hydratationswasser je Mol cyclisches Dipeptid-Enantiomer enthält.

**10.** Verfahren nach einem der Ansprüche 1 bis 9, bei welchem die Reaktion in Gegenwart eines Lösungsmittels ausgeführt wird, in welchem der Aldehyd der Formel II und Cyanwasserstoff löslich sind und welches durch den Katalysator adsorbiert wird.

**11.** Verfahren nach einem der Ansprüche 1 bis 10, bei welchem die Reaktion bei einer Temperatur bei oder unter 0° C ausgeführt wird.

**12.** Verfahren nach einem der Ansprüche 1 bis 11, bei welchem die Reaktion bei einer Temperatur unter -5° C ausgeführt wird.

**13.** Verfahren nach einem der Ansprüche 1 bis 12, bei welchem das cyclische Dipeptid-Enantiomer in einer Menge im Bereich von $10^{-1}$ bis $10^{-6}$ Mol je Mol Aldehyd der Formel II vorliegt.

**14.** Verfahren zur Herstellung eines Pyrethroids, bei welchem ein α-substituiertes α-Cyanomethyl-alkohol-Enantiomer der Formel I, welches nach einem der Ansprüche 1 bis 13 hergestellt worden ist, mit einer Pyrethrin-Säure oder Pyrethroid-Säure oder einem Derivat davon umgesetzt wird.

**15.** Verfahren nach Anspruch 14, bei welchem das α -substituierte α -Cyanomethyl-alkohol-Enantiomer ausgewählt ist aus den Enantiomeren von α-Cyano-3-phenoxybenzyl-alkohol, α-Cyano-4-fluoro-3-phenoxybenzyl - alkohol 3-(2,2-Dichlorovinyloxy)-α-cyanobenzyl-alkohol, α -Cyano-pentafluorobenzylalkohol, α -Cyano-α-(6-phenoxypyrid-2-yl)methyl-alkohol und α-Cyano- α -(5-Benzylfur-2-yl)methyl-alkohol.

**16.** α-substituiertes α-Cyanomethyl-alkohol-Enantiomer der Formel I

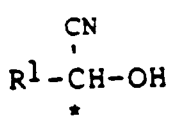

I

worin $R^1$ für eine Gruppe der Formel VIIb

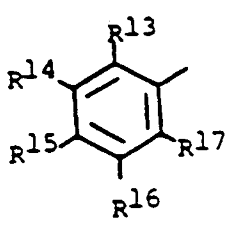

VIIb

steht, worin $R^{13}$, $R^{14}$, $R^{15}$, $R^{16}$, $R^{17}$ unabhängig ausgewählt sind aus Wasserstoff, Halogen, Nitro, Hydroxy, $C_{1-6}$-Alkyl, $C_{1-6}$-Halogenoalkyl, $C_{1-6}$-Hydroxyalkyl, $C_{1-6}$-Alkoxy, ($C_{1-6}$-Alkoxy)-$C_{1-6}$-alkoxy,

Amino, N-($C_{2-6}$-Alkanoyl)amino, N-($C_{1-6}$-Alkylsulfonyl)amino, Sulfamoyl, Ureido, Benzyloxy und Benzoyloxy, wobei zwei benachbarte Substituenten auch eine Buta-1,3-dienylen-Brückengruppebilden können, mit der Maßgabe, daß mindestens einer der Substituenten $R^{13}$, $R^{14}$, $R^{15}$, $R^{16}$ und $R^{17}$ für etwas anderes als Wasserstoff steht.